# EUROPEAN PATENT SPECIFICATION

(11) **EP 1 261 704 B1**
(45) Date of publication and mention of the grant of the patent: **01.07.2009**
(21) Application number: 00993009.0
(22) Date of filing: 29.11.2000
(51) Int. Cl.: C12N 15/12, C07K 14/47, C12N 5/10, C07K 16/30, A61K 48/00, A61K 39/00, G01N 33/50, G01N 33/53, C12Q 1/68

(54) **ISOLATED NUCLEIC ACID MOLECULES ENCODING CANCER ASSOCIATED ANTIGENS, THE ANTIGENS PER SE, AND USES THEREOF**
ISOLIERTE, FÜR KREBS-ASSOZIIERTE ANTIGENE KODIERENDE NUKLEINSÄUREMOLEKÜLE, DIE ANTIGENE PER SE UND DEREN ANWENDUNGEN
MOLECULES D'ACIDES NUCLEIQUES ISOLES CODANT POUR DES ANTIGENES ASSOCIES AU CANCERS, CES ANTIGENES ET LEUR UTILISATION

(30) Priority: 30.11.1999 US 451739; 22.06.2000 US 602362
(43) Date of publication of application: 04.12.2002
(73) Proprietor: LUDWIG INSTITUTE FOR CANCER RESEARCH, New York, New York 10158 (US); MEMORIAL SLOAN-KETTERING CANCER CENTER, New York, NY 10021 (US); CORNELL RESEARCH FOUNDATION, Ithaca, NY 11850 (US)
(72) Inventor: JAGER, Dirk, New York, NY 10021 (US); STOCKERT, Elisabeth, New York, NY 10021 (US); SCANLAN, Matthew, 425 East 68th Street, New York NY10021 (US); KNUTH, Alexander, 60488 Frankfurt am Main (DE); OLD, Lloyd, New York, NY 10158 (US); GURE, Ali, New York, NY 10021 (US); CHEN, Yao-Tseng, New York, NY 10021 (US); JÄGER, Elke, 60488 Frankfurt Am Main (DE)
(74) Representative: Williams, Richard Andrew Norman
(86) International application number: PCT/US2000/042334
(87) International publication number: WO 2001/047959

(56) References cited:
- WO-A-00/60076
- WO-A-00/73801
- WO-A-01/36476
- WO-A-01/75171
- WO-A-94/23728
- WO-A-97/21809
- WO-A-99/04265
- DATABASE EMBL SEQUENCE LIBRARY [Online] 4 August 1999 (1999-08-04) SAITO, A. ,ET AL. : "Homo sapiens mRNA for p33, complete cds. - p24/ING1-ALT1 and p47/ING1-ALT2, distinct alternative transcripts of p33/ING1" XP002190254
- DATABASE EMBL SEQUENCE LIBRARY [Online] 15 November 1999 (1999-11-15) MA, D., ET AL. : "Sequence conservation of ING1 splicing isoforms in divergent species - Homo sapiens p33ING1b (ING1) mRNA, complete cds" XP002190255
- DATABASE TREMBL SEQUENCE LIBRARY [Online] 1 June 1998 (1998-06-01) GARKATSEV, I.A., ET AL./JAEGER, D. ETAL.: "candidate tumour suppressor P33ING1, variant A" XP002190256
- GARKAVTSEV I ET AL: "SUPPRESSION OF THE NOVEL GROWTH INHIBITOR P33ING1 PROMOTES NEOPLASTIC TRANSFORMATION" NATURE GENETICS, NEW YORK, NY, US, vol. 14, December 1996 (1996-12), pages 415-420, XP002948506 ISSN: 1061-4036 cited in the application
- DATABASE EMBL SEQUENCE LIBRARY [Online] 13 November 1999 (1999-11-13) NCI-CGAP: "National Cancer Institute, Cancer Genome Anatomy Project (CGAP), Tumor Gene Index http://www.ncbi.nlm.nih.gov/ncicgap - xj36a10.x1 Soares_NFL_T_GBC_S1 Homo sapiens cDNA clone IMAGE:2659290 3', mRNA sequence" XP002190257
- DATABASE EMBL SEQUENCE LIBRARY [Online] 23 November 1998 (1998-11-23) DU, H., ET AL. : "The sequence of Homo sapiens PAC clone RP5-1048B16 - Homo sapiens PAC clone RP5-1048B16 from 7q34-q36, complete sequence" XP002190258
- TURECI O ET AL: "EXPRESSION OF SSX GENES IN HUMAN TUMORS" INTERNATIONAL JOURNAL OF CANCER, NEW YORK, NY, US, vol. 77, no. 1, 1998, pages 19-23, XP000916544 ISSN: 0020-7136
- TURECI O ET AL: "SEROLOGICAL ANALYSIS OF HUMAN TUMOR ANTIGENS: MOLECULAR DEFINITION AND IMPLICATIONS" MOLECULAR MEDICINE TODAY, ELSEVIER, CAMBRIDGE, GB, vol. 3, no. 8, August 1997 (1997-08), pages 342-349, XP000985587 ISSN: 1357-4310
- GURE A O ET AL: "SSX: A MULTIGENE FAMILY WITH SEVERAL MEMBERS TRANSCRIBED IN NORMAL TESTIS AND HUMAN CANCER" INTERNATIONAL JOURNAL OF CANCER, NEW YORK, NY, US, vol. 72, no. 6, 1997, pages 965-971, XP000916549 ISSN: 0020-7136
- CHEN ET AL: "Identification of multiple cancer/testis antigens by allogenic antibody screening of a melanoma cell line library" PROCEEDINGS OF THE NATIONAL ACADEMY OF SCIENCES OF USA, NATIONAL ACADEMY OF SCIENCE. WASHINGTON, US, vol. 95, June 1998 (1998-06), pages 6919-6923, XP002132946 ISSN: 0027-8424
- SCANLAN ET AL: "Characterization of human colon cancer antigens recognized by autologous antibodies" INTERNATIONAL JOURNAL OF CANCER, NEW YORK, NY, US, vol. 76, no. 5, 29 May 1998 (1998-05-29), pages 652-658, XP002103186 ISSN: 0020-7136 cited in the application
- SAITO A ET AL: "P24/ING1-ALT1 AND P47/ING1-ALT2, DISTINCT ALTERNATIVE TRANSCRIPTS PF P33/ING1" JOURNAL OF HUMAN GENETICS, XX, XX, vol. 45, 2000, pages 177-181, XP000982381
- JAEGER D ET AL: "CANCER-TESTIS ANTIGENS AND ING1 TUMOR SUPPRESSOR GENE PRODUCT ARE BREAST CANCER ANTIGENS: CHARACTERIZATION OF TISSUE-SPECIFIC ING1 TRANSCRIPTS AND A HOMOLOGUE GENE" CANCER RESEARCH, AMERICAN ASSOCIATION FOR CANCER RESEARCH, BALTIMORE, MD, US, vol. 59, 15 December 1999 (1999-12-15), pages 6197-6204, XP000986298 ISSN: 0008-5472
- JAGER DIRK ET AL: "Identification of a tissue-specific putative transcription factor in breast tissue by serological screening of a breast cancer library." CANCER RESEARCH, vol. 61, no. 5, 1 March 2001 (2001-03-01), pages 2055-2061, XP001053562 ISSN: 0008-5472

## Description

This invention relates to antigens associated with cancer, the nucleic acid molecules encoding them, as well as the uses of these.

It is fairly well established that many pathological conditions, such as infections, cancer, autoimmune disorders, etc., are characterized by the inappropriate expression of certain molecules. These molecules thus serve as "markers" for a particular pathological or abnormal condition. Apart from their use as diagnostic "targets", i.e., materials to be identified to diagnose these abnormal conditions, the molecules serve as reagents which can be used to generate diagnostic and/or therapeutic agents. A by no means limiting example of this is the use of cancer markers to produce antibodies specific to a particular marker. Yet another non-limiting example is the use of a peptide which complexes with an MHC molecule, to generate cytolytic T cells against abnormal cells.

Preparation of such materials, of course, presupposes a source of the reagents used to generate these. Purification from cells is one laborious, far from sure method of doing so. Another preferred method is the isolation of nucleic acid molecules which encode a particular marker, followed by the use of the isolated encoding molecule to express the desired molecule.

Two basic strategies have been employed for the detection of such antigens, in e.g., human tumors. These will be referred to as the genetic approach and the biochemical approach. The genetic approach is exemplified by, e.g., dePlaen et al., Proc. Natl. Sci. USA 85: 2275 (1988). In this approach, several hundred pools of plasmids of a cDNA library obtained from a tumor are transfected into recipient cells, such as COS cells, or into antigen-negative variants of tumor cell lines which are tested for the expression of the specific antigen. The biochemical approach, exemplified by, e.g., O. Mandelboim, et al., Nature 369: 69 (1994) is based on acidic elution of peptides which have bound to MHC-class I molecules of tumor cells, followed by reversed-phase high performance liquid chromography (HPLC). Antigenic peptides are identified after they bind to empty MHC-class I molecules of mutant cell lines, defective in antigen processing, and induce specific reactions with cytotoxic T-lymphocytes. These reactions include induction of CTL proliferation, TNF release, and lysis of target cells, measurable in an MTT assay, or a ⁵¹Cr release assay.

These two approaches to the molecular definition of antigens have the following disadvantages: first, they are enormously cumbersome, time-consuming and expensive; and second, they depend on the establishment of cytotoxic T cell lines (CTLs) with predefined specificity.

The problems inherent to the two known approaches for the identification and molecular definition of antigens is best demonstrated by the fact that both methods have, so far, succeeded in defining only very few new antigens in human tumors. See, e.g., van der Bruggen et al., Science 254:1643-1647 (1991); Brichard et al., J. Exp. Med. 178: 489-495 (1993); Coulie, et al.,J. Exp. Med.180: 35-42 (1994); Kawakami, et al., Proc. NatL Acad. Sci. USA 91: 3515-3519 (1994).

Further, the methodologies described rely on the availability of established, permanent cell lines of the cancer type under consideration. It is very difficult to establish cell lines from certain cancer types, as is shown by, e.g., Oettgen, et al., Immunol. Allerg. Clin. North. Am. 10: 607-637 (1990). It is also known that some epithelial cell type cancers are poorly susceptible to CTLs in vitro, precluding routine analysis. These problems have stimulated the art to develop additional methodologies for identifying cancer associated antigens.

One key methodology is described by Sahin, et al., Proc. Natl. Acad. Sci. USA 92: 11810-11913 (1995). Also, see U.S. Patent No. 5,698,396. To summarize, the method involves the expression of cDNA libraries in a prokaryotic host. (The libraries are secured from a tumor sample). The expressed libraries are then immunoscreened with absorbed and diluted sera, in order to detect those antigens which elicit high titer humoral responses. This methodology is known as the SEREX method ("Serological identification of antigens by Recombinant Expression Cloning"). The methodology has been employed to confirm expression ofpreviously identified tumor associated antigens, as well as to detect new ones. See the above referenced patent applications and Sahin, et al., supra, as well as Crew, et al., EMBO J 144: 2333-2340 (1995).

This methodology has been applied to a range of tumor types, including those described by Sahin et al., supra, any Pfreundschuh, supra, as well as to esophageal cancer (Chen et al., Proc. Natl. Acad. Sci. USA 94: 1914-1918 (1997)); lung cancer (Güre et al., Cancer Res. 58: 1034-1041 (1998)); colon cancer (Serial No. 08/948, 705 filed October 10, 1997). Among the antigens identified via SEREX are the SSX2 molecule (Sahin et al., Proc. Natl. Acad. Sci. USA 92 : 11810-11813 (1995); Tureci et al., Cancer Res. 56 : 4766-4772 (1996);NY-ESO-1 Chen, et al., Proc. Natl. Acad. Sci. USA 94 : 1914-1918 (1997) ; and SCP1 (Serial No. 08/892, 705 filed July 15, 1997). Analysis of SEREX identified antigens has shown overlap between SEREX defined and CTL defined antigens. MAGE-1, tyrosinase, and NY-ESO-I have all been shown to be recognized by patient antibodies as well as CTLs, showing that humoral and cell mediated responses do act in concert.

It is clear from this summary that identification of relevant antigens via SEREX is a desirable aim. The inventors have applied this methodology and have identified several new antigens associated with cancer, as detailed in the description which follows.

Accordingly, the present invention provides an isolated cancer associated antigen comprising the amino acid sequence encoded by SEQ ID NO: 15; or which has the amino acid sequence of SEQ ID NO: 16 or SEQ ID NO: 30.

The invention also provides an isolated nucleic acid molecule encoding a cancer associated antigen and comprising the nucleotide sequence of SEQ ID NO: 15, wherein the nucleic acid molecule encodes a polypeptide which has the amino acid sequence of SEQ ID NO: 16 or SEQ ID NO: 30.

The invention further provides an expression vector comprising an isolated nucleic acid molecule as defined herein operably linked to a promoter.

The invention also provides a eukaryotic cell line or prokaryotic cell strain, transformed or transfected with an expression vector as defined herein.

The eukaryotic cell line or prokaryotic cell strain of the invention may also be transfected with a nucleic acid molecule encoding a cytokine. Alternatively or additionally, the eukaryotic cell line or prokaryotic cell strain of the invention may be further transfected by a nucleic acid molecule coding for an MHC molecule. The cytokine is optionally an interleukin; e.g. wherein the interleukin is IL-2, IL-4 or IL-12. A eukaryotic cell line or prokaryotic cell strain of the invention may be a cell line which has been rendered non-proliferative. The eukaryotic cell line may be cell line is a fibroblast cell line. The eukaryotic cell line may be a human cell line.

The invention also provides an expression vector comprising a mutated or attenuated virus and an isolated nucleic acid molecule as defined herein. The virus may be adenovirus or vaccinia virus.

The invention further provides an expression system useful in transfecting a cell, comprising (i) a first vector being a vector as defined herein and (ii) a second vector selected from the group consisting of (a) a vector containing a nucleic acid molecule which codes for an MHC or HLA molecule which presents an antigen derived from said cancer associated antigen and (b) a vector containing a nucleic acid molecule which codes for an interleukin.

The invention also provides an immunogenic composition comprising an isolated cancer antigen as defined herein and a pharmaceutically acceptable adjuvant. The adjuvant may be a cytokine, a saponin, or GM-CSF.

The invention further provides an immunogenic composition comprising at least one peptide consisting of an amino acid sequence of from 8 to 25 amino acids or 8 to 12 amino acids, concatenated to each other in the isolated cancer antigen of claim 1 and a pharmaceutically acceptable adjuvant. The composition may comprise a plurality of peptides which complex with a specific MHC molecule.

The invention also includes a vaccine for treating a subject afflicted with a cancerous condition comprising a eukaryotic cell line as defined herein and a pharmacologically acceptable adjuvant.

The invention further provides an isolated antibody specific for a cancer associated antigen as herein defined. The antibody may be a monoclonal antibody.

The invention also provides a method for monitoring the level of expression of cancer associated antigen as defined herein comprising contacting a sample with a nucleic acid sample. The method may comprise amplifying RNA which codes for the cancer associated antigen as defined herein. The amplification may comprise a polymerase chain reaction.

The invention further provides a method for monitoring the level of cancer associated antigen as defined herein comprising contacting a sample with an antibody as defined herein and determining binding of the antibody to the sample. The antibody may be labelled with a radioactive label or an enzyme. The method may comprise assaying said sample for shed protein.

The invention also provides a method of testing a body fluid sample of a subject for immune cell reactivity to a eukaryotic cell line as defined herein, comprising contacting a eukaryotic cell as defined herein with the sample.

In any of the methods of the invention, the sample is a body fluid or exudates, or a tissue.

### EXAMPLE 1

The SEREX methodology, as described by, e. g. Sahin, et al., Proc. Natl. Acad. Sci. USA 92 : 11810-11813 (19995 ; Chen, et al., Proc. Natl. Acad. Sci. USA 94 : 1914-1918 (1997), and U. S. Patent No. 5, 698, 396. In brief, total RNA was extracted from a sample of a cutaneous metastasis of a breast cancer patient (referred to as "BR11"hereafter), using standard CsCl guanidine thiocyanate gradient methodologies. A cDNA library was then prepared, using commercially available kits designed for this purpose. Following the SEREX methodology referred to supra, this cDNA expression library was amplified, and screened with either autologous BR11 serum which had been diluted to 1: 200, or with allogeneic, pooled serum, obtained from 7 different breast cancer patients, which had been diluted to 1:1000. To carry out the screen, serum samples were first diluted to 1:10, and then preabsorbed with lysates of E. coli that had been transfected with naked vector, and the serum samples were then diluted to the levels described supra. The final dilutions were incubated overnight at room temperature with nitrocellulose membranes containing phage plaques, at a density of 4-5000 plaque forming units ("pfus") per 130 mm plate.

Nitrocellulose filters were washed, and incubated with alkaline phosphatase conjugated, goat anti-human Fcγ secondary antibodies, and reactive phage plaques were visualized via incubation with 5-bromo-4-chloro-3-indolyl phosphate and nitroblue tetrazolium.

This procedure was also carried out on a normal testicular cDNA library, using a 1:200 serum dilution.

A total of 1.12x10⁶ pfus were screened in the breast cancer cDNA library, and 3 8 positive clones were identified. With respect to the testicular library, 4x10⁵ pfus were screened, and 28 positive clones were identified.

Additionally, 8x10⁵ pfus from the BR11 cDNA library were screened using the pooled serum described. Of these, 23 were positive.

The positive clones were subcloned, purified, and excised to forms suitable for insertion in plasmids. Following amplification of the plasmids, DNA inserts were evaluated via restriction mapping (EcoRI-XbaI), and clones which represented different cDNA inserts were sequenced using standard methodologies.

If sequences were identical to sequences found in GenBank, they were classified as known genes, while sequences which shared identity only with ESTs, or were identical to nothing in these data bases, were designated as unknown genes. Of the clones from the breast cancer library which were positive with autologous serum, 3 were unknown genes. Of the remaining 35, 15 were identical to either NY-ESO-1, or SSX2, two known members of the CT antigen family described supra, while the remaining clones corresponded to 14 known genes. Of the testicular library, 12 of the clones were SSX2.

The NY-ESO-1 antigen was not found, probably because the commercial library that was used had been size fractionated to have an average length of 1.5 kilobases, which is larger than full length NY-ESO-1 cDNA which is about 750 base pairs long.

With respect to the screening carried out with pooled, allogeneic sera, four of the clones were NY-ESO-1. No other CT antigens were identified. With the exception of NY-ESO-1, all of the genes identified were expressed universally in normal tissue.

A full listing of the isolated genes, and their frequency of occurrence follows, in tables 1, 2 and 3. Two genes were found in both the BR11 and testicular libraries, i.e., poly (ADP-ribose) polymerase, and tumor suppression gene ING1. The poly (ADP-ribose) polymerase gene has also been found in colon cancer libraries screened via SEREX, as is disclosed by Scanlan, et al., Int. J. Cancer 76: 652-58 (1998) when the genes identified in the screening of the BR11 cDNA library by autologous and allogeneic sera were compared, NY-ESO-1 and human keratin.

**Table 1. SEREX-defined genes identified by autologous screening of BR11 cDNA library**

| Gene group | No. of clones | Comments | Expression |
|---|---|---|---|
| CT genes | 10 | NY-ESO-1 | tumor, testis |
| | 5 | SSX2 | tumor, testis |
| Non-CT genes | 5 | Nuclear Receptor Co-Repressor | ubiquitous |
| | 4 | Poly(ADP-ribose) polymerase | ubiquitous |
| | 2 | Adenylosuccinatelyase | ubiquitous |
| | 2 | cosmid 313 (human) | ESTs: muscle, brain, breast |
| | 1 | CD 151 (transmembrane protein) | ubiquitous |
| | 1 | Human HRY Gen | RT-PCR: multiple normal tissues |
| | 1 | Alanyl-t-RNA-Synthetase | ubiquitous |
| | 1 | NAD(+) ADP-Ribosyltransferase | ubiquitous |
| | 1 | Human keratin 10 | ESTs: multiple normal tissues |
| | 1 | Human EGFR kinase substrate | ubiquitous |
| | 1 | *ING 1* Tumor suppressor gene | RT-PCR: multiple normal tissues |
| | 1 | Unknown gene, | ESTs: pancreas, liver, spleen, uterus |
| | | NCI_CGAP_Pr12 cDNA clone | |
| | 1 | Unknown gene | ESTs: multiple normal tissues |
| | 1 | Unknown gene | RT-PCR: multiple normal tissues |

**Table 2. SEREX-defined genes identified by allogeneic screening of BR11 cDNA library**

| Gene group | No. of clones | Comments | Expression |
|---|---|---|---|
| CT genes | 4 | NY-ESO-1 | tumor, testis |
| | | | |
| Non-CT genes | 6 | zinc-finger helicase | ESTs: brain, fetal heart, total fetus |
| | 4 | Acetoacetyl-CoA-thiolase | ubiquitous |
| | 3 | KIAA0330 gene | ESTs: multiple normal tissues |
| | 2 | U1snRNP | ubiquitous |
| | 1 | Human aldolase A | ubiquitous |
| | 1 | Retinoblastoma binding protein 6 | ESTs: tonsils, fetal brain, endothelial cells, brain |
| | 1 | α2-Macroglobulin receptor associated protein | ubiquitous |
| | 1 | Human Keratin 10 | ESTs: multiple normal tissues |

**Table 3. SEREX-defined genes identified by screening of a testicular cDNA library with BR11 serum**

| Gene group | No. of clones | Comments | Expression |
|---|---|---|---|
| CT genes: | 12 | SSX2 | tumor, testis |
| | | | |
| Non-CT genes: | 3 | Rho-associated coiled-coil forming protein | ubiquitous |
| | 3 | Poly(ADP-ribose) polymerase | ubiquitous |
| | 3 | Gene from HeLa cell, similar to TITIN | ubiquitous |
| | 2 | Gene from parathyroid tumor | RT-PCR: multiple normal tissues |
| | 1 | Transcription termination factor I-interacting peptide 21 | ubiquitous |
| | 1 | Gene from fetal heart | ESTs: multiple normal tissues |
| | 1 | *ING 1* tumor suppressor gene | RT-PCR: multiple normal tissues |
| | 1 | KIAA0647 cDNA | ESTs: multiple normal tissues |
| | 1 | KIAA0667 cDNA | ESTs: multiple normal tissues |

### EXAMPLE 2

The mRNA expression pattern of the cDNAs identified in example 1, in both normal and malignant tissues, was studied. To do this, gene specific oligonucleotide primers were designed which would amplify cDNA segments 300-600 base pairs in length, using a primer melting temperature of 65-70° C. The primers used for amplifying MAGE-1,2,3 and 4, BAGE, NY-ESO-1, SCP1, and SSX1, 2, 3, 4 and 5 were known primers, or were based on published sequences. See Chen, et al. supra; Tureci, et al., Proc. Natl. Acad. Sci. USA 95: 5211-16 (1998). Gure, et al., Int. J. Cancer 72: 965-71 (1997); Chen, et al., Proc. Natl. Acad. Sci. USA 91: 1004-1008 (1994); Gaugler, et al., J. Exp. Med. 179: 921-930 (1994), dePlaen, et al., Immunogenetics 40: 360-369 (1994). RT-PCR was carried out for 35 amplification cycles, at an annealing temp erature of 60°C. Using this RT-PCR assay, the breast cancer tumor specimen was positive for a broad range of CT antigens, including MAGE-1,3 AND 4, BAGE, SSX2, NY-ESO-1 and CT7. The known CT antigens SCP-1, SSX1, 4 and 5 were not found to be expressed.

An additional set of experiments were carried out, in which the seroreactivity of patient sera against tumor antigens was tested. Specially, ELISAs were carried out, in accordance with Stockert, et al., J. Exp. Med. 187: 1349-1354 (1998) to determine if antibodies were present in the patient sera. Assays were run for MAGE-1, MAGE-3, NY-ESO-1, and SSX2. The ELISAs were positive far NY-ESO-1 and SSX2, but not the two MAGE antigens.

### EXAMPLE 3

Two clones (one from the breast cancer cDNA library and one from the testicular library), were identified as a gene referred to as ING1, which is a tumor suppressor gene candidate. See Garkavtsev, et al., Nature 391: 295-8 (1998). The sequence found in the breast cancer library, differed from the known sequence of ING1 at six residues, i.e., positions 818, 836, 855, 861, 866 and 874. The sequence with the six variants is set forth at SEQ ID NO: 1. The sequence of wild type ING1 is set out at SEQ ID NO: 2.

To determine if any of these differences represented a mutation in tumors, a short, PCR fragment which contained the six positions referred to supra was amplified from a panel of allogeneic normal tissue, subcloned, amplified, and sequenced following standard methods.

The results indicated that the sequences in the allogeneic tissues were identical to what was found in tumors, ruling out the hypothesis that the sequence differences were a tumor associated mutation. This conclusion was confirmed, using the testicular library clone, and using restriction analysis of ING1 cDNA taken from normal tissues. One must conclude, therefore, that the sequence information provided by Garkavtsev, et al., supra, is correct.

### EXAMPLE 4

Additional experiments were carried out to determine whether genetic variations might exist in the 5' portion of the ING1 gene, which might differ from the 5' portion of the clone discussed supra (SEQ ID NO: 1). In a first group of experiments, attempts were made to obtain full length ING1 cDNA from both the breast tumor library, and the testicular library. SEQ ID NO: 1 was used as a probe of the library, using standard methods.

Four clones were isolated from the testicular library and none were isolated from the breast cancer library. The four clones, following sequencing, were found to derive from three transcript variants. The three variants were identical from position 586 down to their 3' end, but differed in their 5' regions, suggesting alternatively spliced variants, involving the same exonintron junction. All three differed from the sequence of ING1 described by Garkavtsev, et al., in Nat. Genet.14: 415-420 (1996). These three variants are set out as SEQ ID NOS: 1, 3 and 4.

All of the sequences were then analyzed. The ORFs of SEQ ID NOS: 2, 1 and 4 (SEQ ID NO: 2 is the originally disclosed, ING1 sequence), encode polypeptides of 294,279 and 235 amino acids, of which 233 are encoded by the 3' region common to the three sequences. These putative sequences are set out as SEQ ID NOS: 19, 5, and 7. With respect to SEQ ID NO: 3, however, no translational initiation site could be identified in its 5' region.

### EXAMPLE 5

The data regarding SEQ ID NO: 3, described supra, suggested further experiments to find additional ORFs in the 5-end of variant transcripts of the molecule. In order to determine this, 5'-RACE -PCR was carried out using gene specific and adapted specific primers, together with commercially available products, and standard methodologies.

The primers used for these experiments were:

Cloning and sequencing of the products of RACE PCR showed that the variant sequence of SEQ ID NO: 4 was 5' to SEQ ID NO: 3, and that full length cDNA for the variant SEQ ID NO: 3 contained an additional exon 609 nucleotides long, positioned between SEQ ID NO: 3 and the shared, 3' sequence referred to supra. This exon did not include an ORF. The first available initiation site would be an initial methionine at amino acid 70 of SEQ ID NO: 1. Thus, if expressed, SEQ ID NO: 3 would correspond to a molecule with a 681 base pair, untranslated 5' end and a region encoding 210 amino acids (SEQ ID NO:6).

### EXAMPLE 6

The presence of transcript variants with at least 3 different trancriptional initiation sites, and possibly different promoters, suggested that mRNA expression might be under different, tissue specific regulation.

To determine this, variant-specific primers were synthesized, and RT-PCR was carried out on a panel of tissues, using standard methods.

SEQ ID NO: 1 was found to be expressed universally in all of the normal breast, brain and testis tissues examined, in six breast cancer lines, and 8 melanoma cell lines, and in cultured melanocytes. SEQ ID NO: 3 was found to be expressed in four of the six breast cancer lines, normal testis, liver, kidney, colon and brain. SEQ ID NO: 4 was only found to be expressed by normal testis cells and weakly in brain cells.

### EXAMPLE 7

A further set of experiments were carried out to determine if antibodies against ING1 were present in sera of normal and cancer patients. A phase plaque immuno assay of the type described supra was carried out, using clones of SEQ ID NO: 1 as target. Of 14 allogeneic sera taken from breast cancer patients, two were positive at 1:200 dilutions. All normal sera were negative.

### EXAMPLE 8

The BR11 cDNA library described supra was then screened, using SEQ ID NO: 1 and standard methodologies. A 593 base pair cDNA was identified, which was different from any sequences in the data banks consulted. The sequence of this cDNA molecule is set out at SEQ ID NO: 8.

The cDNA molecule set forth as SEQ ID NO:1 was then used in Southern blotting experiments. In brief, genomic DNA was isolated from normal human tissue, digested with BamHI or Hind III, and then separated onto 0.7% agarose gel, blotted onto nitrocellulose filters, and hybridized using ³²P labelled SEQ ID NO: 1, at high stringency conditions (aqueous buffer, 65 ° C). The probes were permitted to hybridize overnight, and then exposed for autoradiography. Two hybridizing DNA species were identified, i.e., SEQ ID NOS: 1 and 8.

### EXAMPLE 9

The cDNA molecule set forth in SEQ ID NO: 8 was then analyzed. 5'- RACE PCR was carried out using normal fetus cDNA. Full length cDNA for the molecule is 771 base pairs long, without the poly A tail. It shows strong homology to SEQ ID NO: 1, with the strongest homology in the 5' two-thirds (76% identity over nucleotide 1-480); however, the longest ORF is only 129 base pairs, and would encode a poly peptide 42 amino acids long which was homologous to, but much shorter than, the expected expression product of SEQ ID NO: 1.

In addition to the coding region, SEQ ID NO: 8 contains 203 base pairs of 5'-untranslated region, and 439 base pairs of 3'-untranslated region.

RT-PCR assays were carried out, as described supra. All of the normal tissues tested, including brain, colon, testis, tissue and breast, were positive for expression of this gene. Eight melanoma cell lines were tested, of which seven showed varying levels of expression, and one showed no expression. Six breast cancer cell lines were tested, of which four showed various levels of expression, and two showed no expression.

### EXAMPLE 10

An additional breast cancer cDNA library, referred to as "BR17-128", was screened, using autologous sera. A cDNA molecule was identified.

Analysis of the sequence suggested that it was incomplete at the 5' end. To extend the sequence, a testicular cDNA library was screened with a nucleotide probe based upon the partial sequence identified in the breast cancer library. An additional 1200 base pairs were identified following these screenings. The 2011 base pairs of information are set forth in SEQ ID NO: 15.

The longest open reading frame is 1539 base pairs, corresponding to a protein of about 59.15 kilodaltons. The deduced sequence is set forth at SEQ ID NO: 16.

RT-PCR was then carried out using the following primers:

Strong signals were observed in normal testis and breast tissue, and weak expression was observed in placenta.

No expression was found in normal brain, kidney, liver, colon, adrenal, fetal brain, lung, pancreas, prostate, thymus, uterus, and ovary tissue of tumor cell lines tested, 2 of the breast cancer lines were strongly positive and two were weakly positive. Of melanoma two of 8 were strongly positive, and 3 were weakly positive. Of lung cancer cell lines, 4 of 15 were strongly positive, and 3 were weakly positive.

When cancer tissue specimens were tested, 16 of 25 breast cancer samples were strongly positive, and 3 additional samples were weakly positive. Two of 36 melanoma samples were positive (one strong, one weak). All other cancer tissue samples were negative.

When Northern blotting was carried out, a high molecular weight smear was observed in testis, but in no other tissues tested.

### EXAMPLE 11

Further experiments were carried out using the tumor sample referred to in example 10, supra. This sample was derived from a subcutaneous metastasis of a 60 year old female breast cancer patient. Total RNA was extracted, as described supra. Following the extraction, a cDNA library was constructed in λ-ZAP expression vectors, also as described supra. Screening was carried out, using the protocol set forth in example 1. A total of 7 x 10⁵ pfus were screened. Fourteen reactive clones were identified, purified, and sequenced. The sequences were then compared to published sequences in GenBank and EST databases. These analyses indicated that the clones were derived from seven distinct genes, two ofwhich were known, and five unknown. The two known genes were "PBK-1" (three clones), and TI-227 (one clone). These are universally expressed genes, with the libraries referred to supra showing ESTs for these genes from many different tissues.

With respect to the remaining 10 clones, six were derived from the same gene, referred to hereafter as "NY-BR-1." Three cDNA sequences were found in the EST database which shared identity with the gene. Two of these (AI 951118 and AW 373574) were identified as being derived from a breast cancer library, while the third (AW 170035), was from a pooled tissue source.

### EXAMPLE 12

The distribution of the new gene NY-BR-1 referred to supra was determined via RT-PCR. In brief, gene specific oligonucleotide NY-BR-1 primers were designed to amplify cDNA segments 300-600 base pairs in length, with primer melting temperatures estimated at 65-70°C.

The RT-PCR was then carried out over 30 amplification cycles, using a thermal cycler, and an annealing temperature of 60°C. Products were analyzed via 1.5% gel electrophoresis, and ethidium bromide visualization. Fifteen normal tissues (adrenal gland, fetal brain, lung, mammary gland, pancreas, placenta, prostate, thymus, uterus, ovary, brain, kidney, liver, colon and testis) were assayed. The NY-BR-1 clone gave a strong signal in mammary gland and testis tissue, and a very faint signal in placenta. All other tissues were negative. The other clones were expressed universally, based upon comparison to information in the EST database library, and were not pursued further.

The expression pattern of NY-BR-1 in cancer samples was then tested, by carrying out RT-PCR, as described supra, on tumor samples.

In order to determine the expression pattern, primers:
caaagcagag cctcccgaga ag (SEQ ID NO: 20) and
cctatgctgc tcttcgattc ttcc (SEQ ID NO: 21) were used.
Of twenty-five breast cancer samples tested, twenty two were positive for NY-BR-1. Of these, seventeen gave strong signals, and five gave weak to modest signals.

An additional 82 non-mammary tumor samples were also analyzed, divided into 36 melanoma, 26 non small cell lung cancer, 6 colon cancer, 6 squamous cell carcinoma, 6 transitional cell carcinoma, and two leiyomyosarcomas. Only two melanoma samples were positive for NY-BR-1 expression.

The study was then extended to expression of NY-BR-1 in tissue culture. Cell lines derived from breast tumor, melanoma, and small cell lung cancer were studied. Four of six breast cancer cells were positive (two were very weak), four of eight melanoma (two very weak), and seven of fourteen small cell lung cancer lines (two very weak) were positive.

### EXAMPLE 13

In order to determine the complete cDNA molecule for NY-BR-1, the sequences of the six clones referred to supra were compiled, to produce a nucleotide sequence 1464 base pairs long. Analysis of the open reading frame showed a continuous ORF throughout, indicating that the compiled sequence is not complete.

Comparison of the compiled sequence with the three EST library sequences referred to supra allowed for extension of the sequence. The EST entry AW170035 (446 base pairs long) overlapped the compiled sequence by 89 base pairs at its 5' end, permitting extension of the sequence by another 357 base pairs. A translational terminal codon was identified in this way, leading to a molecule with a 3'-untranslated region 333 base pairs long. The 5' end of the molecule was lacking, however, which led to the experiment described infra.

### EXAMPLE 14

In order to determine the missing, 5' end of the clone described supra, a commercially available testis cDNA expression library was screened, using a PCR expression product of the type described supra as a probe. In brief, 5x10⁴ pfus per 150 mm plate were transferred to nitrocellulose membranes, which were then submerged in denaturation solution (1.5M NaCl and 0.5 M NaOH), transferred to neutralization solution (1.5 M NaCl and 0.5M Tris-HCl), and then rinsed with 0.2M Tris-HCl, and 2xSSC. Probes were labelled with ³²P and hybridization was carried out at high stringency conditions (i.e., 68°C, aqueous buffer). Any positive clones were subcloned, purified, and in vivo excised to plasmid PBK-CMV, as described supra.

One of the clones identified in this way included an additional 1346 base pairs at the 5' end; however, it was not a full length molecule. A 5'-RACE-PCR was carried out, using commercially available products. The PCR product was cloned into plasmid vector pGEMT and sequenced. The results indicated that cDNA sequence was extended 1292 base pairs further, but no translation initiation site could be determined, because no stop codons could be detected. It could be concluded, however, that the cDNA of the NY-BR17 clone comprises at least 4026 nucleotides, which are presented as SEQ ID NO: 22. The molecule, as depicted, encodes a protein at least about 152.8 kDA in molecular weight. Structurally, there are 99 base pairs 5' to the presumed translation initiation site, and an untranslated segment 333 base pairs long at the 3' end. The predicted amino acid sequence of the coding region for SEQ ID NO: 22 is set out at SEQ. ID NO: 23.

SEQ ID NO: 23 was analyzed for motifs, using the known search programs PROSITE and Pfam. A bipartite nuclear localization signal motif was identified at amino acids 17-34, suggesting that the protein is a nuclear protein. Five tandem ankyrin repeats were identified, at amino acids 49-81, 82-114, 115-147, 148-180 and 181-213. A bZIP site (i.e. a DNA binding site followed by a leucine zipper motif) was found at amino acid positions 1077-1104, suggesting a transcription factor function. It was also observed that three repetitive elements were identified in between the ankyrin repeats and the bZIP DNA binding site. To elaborate, a repetitive element 117 nucleotides long is trandemly repeated 3 times, between amino acids 459-815. The second repetitive sequence, consisting of 11 amino acids, repeats 7 times between amino acids 224 and 300. The third repetitive element, 34 amino acids long, is repeated twice, between amino acids 301-368.

### EXAMPLE 15

The six clones described supra were compared, and analysis revealed that they were derived from two different splice variants. Specifically, two clones, referred to as "BR17-8" and "BR 17-44a", contain one more exon, of 111 base pairs (nucleotides 3015-3125 of SEQ ID NO: 22), which encodes amino acids 973-1009 of SEQ ID NO: 23, than do clones BR 17-1a, BR17-35b and BR17-44b. The shortest of the six clones, BR17-128, starts 3' to the additional exons. The key structural elements referred to supra were present in both splice variants, suggesting that there was no difference in biological function.

The expression pattern of the two splice variants was assessed via PT-PCR, using primers which spanned the 111 base pair exon referred to supra.

The primers used were:
aatgggaaca agagctctgc ag (SEQ ID NO: 24) and
gggtcatctg aagttcagca ttc (SEQ ID NO: 25)
Both variants were expressed strongly in normal testis and breast. The longer variant was dominant in testis, and the shorter variant in breast cells. When breast cancer cells were tested, co-typing of the variant was observed, (7 strongly, 2 weakly positive, and 1 negative), with the shorter variant being the predominant form consistently.

### EXAMPLE 16

The frequency of antibody response against NY-BR-1 in breast cancer patients was tested. To do this, a recombinant protein consisting of amino acids 993-1188 of SEQ ID NO: 23 was prepared. (This is the protein encoded by clone BR 17-128, referred to supra). A total of 140 serum samples were taken from breast cancer patients, as were 60 normal serum samples. These were analyzed via Western blotting, using standard methods.

Four of the cancer sera samples were positive, including a sample from patient BR17. All normal sera were negative.

An additional set of experiments was then carried out to determine if sera recognized the portion of NY-BR-1 protein with repetitive elements. To do this, a different recombinant protein, consisting of amino acids 405-1000 was made, and tested in Western blot assays. None of the four antibody positive sera reacted with this protein indicating that an antibody epitope is located in the non-repetitive, carboxy terminal end of the molecule.

### EXAMPLE 17

The screening of the testicular cDNA library referred to supra resulted, inter alia, in the identification of a cDNA molecule that was homologous to NY-BR-1. The molecule is 3673 base pairs in length, excluding the poly A tail. This corresponded to nucleotides 1-3481 of SEQ ID NO: 22, and showed 62% homology thereto. No sequence identity to sequences in libraries was noted. ORF analysis identified an ORF from nucleotide 641 through the end of the sequence, with 54% homology to the protein sequence of SEQ. ID NO: 23. The ATG initiation codon of this sequence is 292 base pairs further 3' to the presumed initiation codon of NY-BR-1, and is preceded by 640 untranslated base pairs at its 5' end. This 640 base pair sequence includes scattered stop codons. The nucleotide sequence and deduced amino acid sequence are presented as SEQ ID NOS: 26 and 27, respectively.

RT-PCR analysis was carried out in the same way as is described supra, using primers:
tct catagat gctggtgctg atc (SEQ ID NO: 28) and
cccagacatt gaattttggc agac (SEQ ID NO: 29).
Tissue restricted mRNA expression was found. The expression pattern differed from that of SEQ ID NO: 22. In brief, of six normal tissues examined, strong signals were found in brain and testis only. There was no or weak expression in normal breast tissues, and kidney, liver and colon tissues were negative. Eight of ten 10 breast cancer specimens tested supra were positive for SEQ. ID NO: 26. Six samples were positive for both SEQ. ID NO: 22 and 26, one for SEQ. ID NO: 22 only, two for the SEQ. ID NO: 26 only, and one was negative for both.

### EXAMPLE 18

Recently, a working draft of the human genome sequence was released. This database was searched, using standard methods, and NY-BR-1 was found to have sequence identity with at least three chromosome 10 clones, identified by Genbank accession numbers AL157387, AL37148, and AC067744. These localize NY-BR-1 to chromosome 10 p11.21-12.1.

The comparison of NY-BR-1 and the human genomic sequence led to definition of NY-BR-1 exon-intron organization. In brief, the coding region of the gene contains essentially 19 structurally distinct exons with at least 2 exons encoding 3' untranslated regions. Detailed exon-intron junction information is described at Genbank AF 269081.

The six ankyrin repeats, referred to supra, are all found within exon 7. The 357 nucleotide repeating unit is composed of exons 10-15. The available genomic sequences are not complete, however, and only one of the three copies was identified, suggesting that DNA sequences between exons 5 and 10 may be duplicated and inserted in tandem, during genetic evolution. In brief, when the isolated NY-BR-1 cDNA clone was analyzed, three complete and one incomplete copy of the repeating units are present. The exon sequences can be expresses as exons 1-2-3-4-5-6-7-8-9-(10-11-12-13-14-15)-(10A-11A-12A-13A-14A-15A)-(10B-11B-12B-13B-14B-15B)-(10C-11C-12C-13C-14C)-16-17-18-19-20-21, wherein A, B & C are inexact copies of exon 10-15 sequences. Cloned, NY-BR-1 cDNA has 38 exons in toto.

It was noted, supra, that the sequence of NY-BR-1 cDNA was not complete at the 5' end. Genonic sequence (Genbank AC067744), permitted extension of the 5' end. Translation of the 5' genonic sequence led to the identification of a new translation initiation site, 168 base pairs upstream of the previously predicted ATG initiation codon. This led to an NY-BR-1 polypeptide including 1397 amino acid longer, 56 residue of which are added at the N-terminus, compared to prior sequence information, i.e.:
MEEISAAAVKWPGPERPSPFSQLVYTSNDSYIVHSGDLRKIHKAASRGQVRKLEK(SEQ ID NO: 30).

### EXAMPLE 20

Reference was made, supra, to the two difference splice variants of NY-BR-1. Comparison of the splice variants with the genomic sequence confirmed that an alternate splicing event, with the longer variant incorporating part of intron 33 into exon 34 (i.e., exon 17 of the basic exon/intron framework described supra).

Key structural elements that were predicted in NY-BR-1, described supra, are present in both variants, suggesting that there is no difference in biological function, or subcellular location.

### EXAMPLE 21

As with NY Bur-1, the variant NY-BR-1.1, described supra, was screened against the working draft of the human genome sequence. One clone was found with sequence identity, i.e., GenBank AL359312, derive from chromosome 9. Thus, NY-BR-1 and NY-BR-1.1 both appear to be functioning genes, on two different chromosomes. The Genbank sequence referred to herein does not contain all of NY-BR-1.1, which precludes defining exon-intron structure. Nonetheless, at least 3 exons can be defined, which correspond to exons 16-18 of the NY-BR-1 basic framework. Exon-intron junctions are conserved.

### EXAMPLE 22

A series ofpeptides were synthesized, based upon the amino acid sequence of NY-BR-1, as set forth in SEQ ID NO: 23. These were then tested for their ability to bind to HLA-A2 molecules and to stimulate CTL proliferation, using an ELISPOT assay. This assay involved coating 96-well, flat bottom nitrocellulose plates with 5ug/ml of anti-interferon gamma antibodies in 100 ul of PBS per well, followed by overnight incubation. Purified CD8⁺ cells, which had been separated from PBL samples via magnetic beads coated with anti-CD8 antibodies were then added, at 1x10⁵ cells/well, in RPMI 1640 medium, that had been supplemented with 10% human serum, L-asparagine (50 mg/l), L-arginine (242 mg/l), L-glutamine (300 mg/l), together with IL-2 (2.5ng/ml), in a final volume of 100 ul. CD8⁺ effector cells were prepared by presensitizing with peptide, and were then added at from 5x10³ to 2x10⁴ cells/well. Peptides were pulsed onto irradiated T2 cells at a concentration of 10ug/ml for 1 hour, washed and added to effector cells, at 5x10⁴ cells/well. The plates were incubated for 16 hours at 37°C, washed six times with 0.05% Tween 20/PBS, and were then supplemented with biotinylated, anti-interferon gamma specific antibody at 0.5 ug/ml. After incubation for 2 hours at 37°C, plates were washed, and developed with commercially available reagents, for 1 hour, followed by 10 minutes of incubation with dye substrate. Plates were then prepped for counting, positives being indicated by blue spots. The number of blue spots/well was determined as the frequency of NY-ESO-1 specific CTLs/well.

Experiments were run, in triplicate, and total number of CTLs was calculated. As controls, one of reagents alone, effector cells alone, or antigen presenting cells alone were used. The difference between the number of positives in stimulated versus non-stimulated cells, was calculated as the effective number of peptide specific CTLs above background. Three peptides were found to be reactive, i.e.:
LLSHGAVIEV (amino acids 102-111 of SEQ ID NO: 23)
SLSKILDTV (amino acids 904-912 of SEQ ID NO: 23)
SLDQKLFQL (amino acids 1262-1270 of SEQ ID NO: 23).

The complete list of peptides tested, with reference to their position in SEQ ID NO: 23, follows:

| Peptide | Position |
|---|---|
| FLVDRKVCQL | 35-43 |
| ILIDSGADI | 68-76 |
| AVYSEILSV | 90-98 |
| ILSVVAKLL | 95-103 |
| LLSHGAVIEV | 102-111 |
| KLLSHGAVI | 101-109 |
| FLLIKNANA | 134-142 |
| MLLQQNVDV | 167-175 |
| GMLLQQNVDV | 166-175 |
| LLQQNVDVFA | 168-177 |
| IA WEKKETPV | 361-370 |
| SLFESSAKI | 430-438 |
| CIPENSIYQKV | 441-450 |
| KVMEINREV | 449-457 |
| ELMDMQTFKA | 687-696 |
| ELMDMQTFKA | 806-815 |
| SLSKILDTV | 904-912 |
| KILDTVHSC | 907-915 |
| ILNEKIREEL | 987-996 |
| RIQDIELKSV | 1018-1027 |
| YLLHENCML | 1043-1051 |
| CMLKKEIAML | 1049-1058 |
| AMLKLELATL | 1056-1065 |
| KILKEKNAEL | 1081-1090 |
| VLIAENTML | 1114-1122 |
| CLQRKMNVDV | 1174-1183 |
| KMNVDVSST | 1178-1186 |
| SLDQKLFQL | 1262-1270 |
| KLFQLQSKNM | 1266-1275 |
| FQLQSKNMWL | 1268-1277 |
| QLQSKNMWL | 1269-1277 |
| NMWLQQQLV | 1274-1282 |
| WLQQQLVHA | 1276-1284 |
| KITIDIHFL | 1293-1301 |

The foregoing examples describe the isolation of a nucleic acid molecule which encodes a cancer associated antigen. "Associated" is used herein because while it is clear that the relevant molecule was expressed by several types of cancer, other cancers, not screened herein, may also express the antigen.

It is to be understood that all sequences which encode the claimed antigen are a part of the invention.

Proteins, polypeptides and peptides of the invention may, but do not necessarily provoke CTL responses when complexed with an HLA molecule, such as an HLA-A2 molecule. They may also bind to different MHC orHLA molecules, including, but notbeing limited to, HLA-A1, A2, A3, B7, B8, Cw3, Cw6, or serve, e.g., as immunogens, as part of immunogenic cocktail compositions, where they are combined with other proteins or polypeptides, and so forth.

Also a part of the invention are expression vectors which incorporate the nucleic acid molecules of the invention, in operable linkage (i.e., "operably linked") to a promoter. Construction of such vectors, such as viral (e.g., adenovirus or Vaccinia virus) or attenuated viral vectors is well within the skill of the art, as is the transformation or transfection of cells, to produce eukaryotic cell lines, or prokaryotic cell strains which encode the molecule of interest. Exemplary of the host cells which can be employed in this fashion are COS cells, CHO cells, yeast cells, insect cells (e.g., Spodoptera frugiperda), NIH 3T3 cells, and so forth. Prokaryotic cells, such as E. coli and other bacteria may also be used. Any of these cells can also be transformed ortransfectedwith further nucleic acid molecules, such as those encoding cytokines, e.g., interleukins such as IL-2, 4, 6, or 12 or HLA or MHC molecules.

Also a part of the invention are the antigens as claimed, both in original form and in any different post translational modified forms. The molecules are large enough to be antigenic without any posttranslational modification, and hence are useful as immunogens, when combined with an adjuvant (or without it), in both precursor and post-translationally modified forms. Antibodies produced using these antigens, both poly and monoclonal, are also a part of the invention as well as hybridomas which make monoclonal antibodies to the antigens. The whole protein can be used therapeutically, or in portions, as discussed infra. Also a part of the invention are antibodies against this antigen, be these polyclonal, monoclonal, reactive fragments, such as Fab, (F(ab)₂' and other fragments, as well as chimeras, humanized antibodies, recombinantly produced antibodies, and so forth.

As is clear from the disclosure, one may use the proteins and nucleic acid molecules of the invention diagnostically. The SEREX methodology discussed herein is premised on an immune response to a pathology associated antigen. Hence, one may assay for the relevant pathology via, e.g., testing a body fluid sample of a subject, such as serum, for reactivity with the antigen per se. Reactivity would be deemed indicative ofpossible presence of the pathology. So, too, could one assay for the expression of any of the antigens via any of the standard nucleic acid hybridization assays which are well known to the art, and need not be elaborated upon herein. One could assay for antibodies against the subject molecules, using standard immunoassays as well.

Analysis of SEQ ID NO: 15, will show that there are 5' and 3' non-coding regions presented therein. The invention relates to those isolated nucleic acid molecules which contain at least the coding segment, and which may contain any or all of the non-coding 5' and 3' portions.

Described herein are portions of the relevant nucleic acid molecules which can be used, for example, as oligonucleotide primers and/or probes, such as one or more of SEQ ID NOS: 9,10,11, 12, 3,14,17,18,20, 21, 24, 25, 28, and 29 as well as amplification products like nucleic acid molecules comprising at least nucleotides 305-748 of SEQ ID NO: 1, or amplification products described in the examples, including those in examples 12, 14, etc.

As was discussed supra, study of other members of the "CT" family reveals that these are also processed to peptides which provoke lysis by cytolytic T cells. There has been a great deal of work on motifs for various MHC or HLA molecules, which is applicable here. Described herein is a therapeutic method, wherein one or more peptides derived from the antigens of the invention which bind to an HLA molecule on the surface of a patient's tumor cells are administered to the patient, in an amount sufficient for the peptides to bind to the MHC/HLA molecules, and provoke lysis by T cells. Any combination of peptides may be used. These peptides, which may be used alone or in combination, as well as the entire protein or immunoreactive portions thereof, may be administered to a subject in need thereof, using any of the standard types of administration, such as intravenous, intradermal, subcutaneous, oral, rectal, and transdermal administration. Standard pharmaceutical carriers, adjuvants, such as saponins, GM-CSF, and interleukins and so forth may also be used. Further, these peptides and proteins may be formulated into vaccines with the listed material, as may dendritic cells, or other cells which present relevant MHC/peptide complexes.

Also described herein are therapies, wherein nucleic acid molecules which encode the proteins of the invention, one or more or peptides which are derived from these proteins are incorporated into a vector, such as a Vaccinia or adenovirus based vector, to render it transfectable into eukaryotic cells, such as human cells. Similarly, nucleic acid molecules which encode one or more of the peptides may be incorporated into these vectors, which are then the major constituent of nucleic acid bases therapies.

Any of these assays can also be used in progression/regression studies. One can monitor the course of abnormality involving expression of these antigens simply by monitoring levels of the protein, its expression, antibodies against it and so forth using any or all of the methods set forth supra.

It should be clear that these methodologies may also be used to track the efficacy of a therapeutic regime. Essentially, one can take a baseline value for a protein of interest using any of the assays discussed supra, administer a given therapeutic agent, and then monitor levels of the protein thereafter, observing changes in antigen levels as indicia of the efficacy of the regime.

As was indicated supra, the inventors recognised, inter alia, an "integrated" immune response to the molecules of the invention. One ramification ofthis is the ability to monitor the course of cancer therapy. In this method, which is a part of the invention, a subject in need of the therapy receives a vaccination of a type described herein. Such a vaccination results, e.g., in a T cell response against cells presenting HLA/peptide complexes on their cells. The response also includes an antibody response, possibly a result of the release of antibody provoking proteins via the lysis of cells by the T cells. Hence, one can monitor the effect of a vaccine, by monitoring an antibody response. As is indicated, supra, an increase in antibody titer may be taken as an indicia of progress with a vaccine, and vice versa. Hence, a further application of the invention is a method for monitoring efficacy of a vaccine, following administration thereof by determining levels of antibodies in the subject which are specific for the vaccine itself, or a large molecule of which the vaccine is a part.

The identification of the subj ect proteins as being implicated in pathological conditions such as cancer also suggests a number of therapeutic approaches in addition to those discussed supra. The experiments set forth supra establish that antibodies are produced in response to expression of the protein. Hence, further described herein is the treatment of conditions which are characterized by aberrant or abnormal levels of one or more of the proteins, via administration of antibodies, such as humanized antibodies, antibody fragments, and so forth. These may be tagged or labelled with appropriate cystostatic or cytotoxic reagents.

T cells may also be administered. It is to be noted that the T cells may be elicited in vitro using immune responsive cells such as dendritic cells, lymphocytes, or any other immune responsive cells, and then reperfused into the subject being treated.

Note that the generation of T cells and/or antibodies can also be accomplished by administering cells, preferably treated to be rendered non-proliferative, which present relevant T cell or B cell epitopes for response, such as the epitopes discussed supra.

The therapeutic approaches may also include antisense therapies, wherein an antisense molecule, preferably from 10 to 100 nucleotides in length, is administered to the subject either "neat" or in a carrier, such as a liposome, to facilitate incorporation into a cell, followed by inhibition of expression of the protein. Such antisense sequences may also be incorporated into appropriate vaccines, such as in viral vectors (e.g., Vaccinia), bacterial constructs, such as variants of the known BCG vaccine, and so forth.
## Claims

1. An isolated cancer associated antigen comprising the amino acid sequence encoded by SEQ ID NO: 15; or which has the amino acid sequence of SEQ ID NO: 16 or SEQ ID NO: 30.

2. An isolated nucleic acid molecule encoding a cancer associated antigen and comprising the nucleotide sequence of SEQ ID NO: 15, wherein the nucleic acid molecule encodes a polypeptide which has the amino acid sequence of SEQ ID NO: 16 or SEQ ID NO: 30.

3. An expression vector comprising an isolated nucleic acid molecule of claim 2, operably linked to a promoter.

4. A eukaryotic cell line or prokaryotic cell strain, transformed or transfected with an expression vector of claim 3.

5. A eukaryotic cell line or prokaryotic cell strain as claimed in claim 4, wherein the cell line is also transfected with a nucleic acid molecule encoding a cytokine.

6. A eukaryotic cell line or prokaryotic cell strain as claimed in claim 4 or claim 5, wherein said cell line is further transfected by a nucleic acid molecule coding for an MHC molecule.

7. A eukaryotic cell line or prokaryotic cell strain as claimed in claim 5 or claim 6, wherein said cytokine is an interleukin.

8. A eukaryotic cell line or prokaryotic cell strain as claimed in claim 7, wherein the interleukin is IL-2, IL-4 or IL-12.

9. A eukaryotic cell line or prokaryotic cell strain as claimed in any of claims 5 to 8, wherein the cell line has been rendered non-proliferative.

10. A eukaryotic cell line as claimed in any of claims 5 to 9, wherein the cell line is a fibroblast cell line.

11. A eukaryotic cell line as claimed in any of claims 4 to 10 which is a human cell line.

12. An expression vector comprising a mutated or attenuated virus and an isolated nucleic acid molecule of claim 2.

13. An expression vector as claimed in claim 12, wherein said virus is adenovirus or vaccinia virus.

14. An expression vector as claimed in claim 13, wherein the virus is vaccinia virus.

15. An expression vector as claimed in claim 13, wherein the virus is adenovirus.

16. An expression system useful in transfecting a cell, comprising (i) a first vector being a vector of claim 3 and (ii) a second vector selected from the group consisting of (a) a vector containing a nucleic acid molecule which codes for an MHC or HLA molecule which presents an antigen derived from said cancer associated antigen of claim 1 and (b) a vector containing a nucleic acid molecule which codes for an interleukin.

17. An immunogenic composition comprising an isolated cancer antigen of claim 1, and a pharmaceutically acceptable adjuvant.

18. An immunogenic composition comprising at least one peptide consisting of an amino acid sequence of from 8 to 25 amino acids or 8 to 12 amino acids, concatenated to each other in the isolated cancer antigen of claim 1 and a pharmaceutically acceptable adjuvant.

19. An immunogenic composition as claimed in claim 17 or claim 18, wherein the adjuvant is a cytokine, a saponin, or GM-CSF.

20. An immunogenic composition as claimed in claim 19, wherein the composition comprises a plurality of peptides which complex with a specific MHC molecule.

21. A vaccine for treating a subject afflicted with a cancerous condition comprising a eukaryotic cell line of any of claims 4 to 11 and a pharmacologically acceptable adjuvant.

22. An isolated antibody specific for a cancer associated antigen of claim 1.

23. An isolated antibody as claimed in claim 22, wherein the antibody is a monoclonal antibody.

24. A method for monitoring the level of expression of the cancer associated antigen of claim 1, comprising contacting a sample with a nucleic acid molecule of claim 2 and determining hybridization of the nucleic acid with the said sample.

25. A method as claimed in claim 24, comprising amplifying RNA which codes for the cancer associated antigen of claim 1.

26. A method as claimed in claim 25, wherein the amplification comprises a polymerase chain reaction.

27. A method for monitoring the level of cancer associated antigen of claim 1 comprising contacting a sample with an antibody of claim 22 or claim 23, and determining binding of the antibody to the sample.

28. A method as claimed in claim 27, wherein the antibody is labelled with a radioactive label or an enzyme.

29. A method as claimed in claim 27 or claim 28, comprising assaying said sample for shed protein.

30. A method of testing a body fluid sample of a subject for immune cell reactivity to a eukaryotic cell line of any of claims 4 to 11, comprising contacting a eukaryotic cell of any of claims 4 to 11 with the sample.

31. A method as claimed in any of claims 24 to 30, wherein said sample is a body fluid or exudates.

32. A method as claimed in any of claims 24 to 30, wherein said sample is a tissue.

## Patentansprüche

1. Isoliertes Krebs assoziiertes Antigen, das die durch SEQ ID NO: 15 codierte Aminosäuresequenz umfasst; oder das die Aminosäuresequenz SEQ ID NO:16 oder SEQ ID NO: 30 aufweist.

2. Isoliertes Nukleinsäuremolekül, das ein Krebs assoziierte Antigen codiert und die Nukleotidsequenz SEQ ID NO: 15 umfasst, wobei das Nukleinsäuremolekül ein Polypeptid codiert, das die Aminosäuresequenz SEQ ID NO: 16 oder SEQ ID NO:30 aufweist.

3. Expressionsvektor, der ein isoliertes Nukleinsäuremolekül nach Anspruch 2 umfasst, operativ verknüpft mit einem Promotor.

4. Eukaryotische Zelllinie oder pokaryotischer Zellstrang, transformiert oder transfiziert mit einem Expressionsvektor nach Anspruch 3.

5. Eukaryotische Zelllinie oder pokaryotischer Zellstrang nach Anspruch 4, wobei die Zelllinie auch mit einem Nukleinsäuremolekül transfiziert ist, das ein Zytokin codiert.

6. Eukaryotische Zelllinie oder pokaryotischer Zellstrang nach Anspruch 4 oder Anspruch 5, wobei die genannte Zelllinie ferner durch eine Nukleinsäuremolekülcodierung für ein MHZ-Molekül transfiziert ist.

7. Eukaryotische Zelllinie oder pokaryotischer Zellstrang nach Anspruch 5 oder Anspruch 6, wobei es sich bei dem genannten Zytokin um ein Interleukin handelt.

8. Eukaryotische Zelllinie oder pokaryotischer Zellstrang nach Anspruch 7, wobei es sich bei dem Interleukin um IL-2, IL-4 oder IL-12 handelt.

9. Eukaryotische Zelllinie oder pokaryotischer Zellstrang nach einem der Ansprüche 5 bis 8, wobei die Zelllinie nicht-proliferativ gestaltet ist.

10. Eukaryotische Zelllinie nach einem der Ansprüche 5 bis 9, wobei es sich bei der Zelllinie um eine Fibroblast-Zelllinie handelt.

11. Eukaryotische Zelllinie nach einem der Ansprüche 4 bis 10, wobei es sich dabei um eine humane Zelllinie handelt.

12. Expressionsvektor, der ein mutiertes oder abgeschwächtes Virus und ein isoliertes Nukleinsäuremolekül nach Anspruch 2 umfasst.

13. Expressionsvektor nach Anspruch 12, wobei das genannte Virus ein Adenovirus oder Vaccinia Virus darstellt.

14. Expressionsvektor nach Anspruch 13, wobei das Virus ein Vaccinia Virus darstellt.

15. Expressionsvektor nach Anspruch 13, wobei das Virus ein Adenovirus Virus darstellt.

16. Expressionssystem, das zum Transfizieren einer Zelle nützlich ist, wobei das System folgendes umfasst: (i) einen ersten Vektor, bei dem es sich um den Vektor aus Anspruch 3 handelt; und (ii) einen zweiten Vektor, der aus der Gruppe ausgewählt wird, die folgendes umfasst (a) einen Vektor, der ein Nukleinsäuremolekül aufweist, das für ein MHC- oder HLA-Molekül codiert, das ein von dem genannten Krebs assoziierten Antigen nach Anspruch 1 abgeleitetes Antigen darstellt; und (b) einen Vektor, der ein Nukleinsäuremolekül enthält, das für ein Interleukin codiert.

17. Immunogene Zusammensetzung, die ein isoliertes Krebsantigen nach Anspruch 1 und ein pharmazeutisch akzeptables Adjuvans umfasst.

18. Immunogene Zusammensetzung, die mindestens ein Peptid umfasst, das aus einer Aminosäuresequenz aus 8 bis 25 Aminosäuren oder 8 bis 12 Aminosäuren besteht, miteinander verkettet in dem isolierten Krebsantigen nach Anspruch 1 und einem pharmazeutisch akzeptablen Adjuvans.

19. Immunogene Zusammensetzung nach Anspruch 17 oder Anspruch 18, wobei es sich bei dem Adjuvans um ein Zytokin, ein Saponin oder GM-CSF handelt.

20. Immunogene Zusammensetzung nach Anspruch 19,wobei die Zusammensetzung eine Mehrzahl von Peptiden umfasst, die verflochten sind mit einem spezifischen MHC-Molekül.

21. Vakzine zur Behandlung eines an einem Krebszustand leidenden Patienten, wobei die Vakzine eine eukaryotische Zelllinie nach einem der Ansprüche 4 bis 11 und ein pharmazeutisch akzeptables Adjuvans umfasst.

22. Isolierter Antikörper, der für ein Krebs assoziiertes Antigen nach Anspruch 1 spezifisch ist.

23. Isolierter Antikörper nach Anspruch 22, wobei es sich bei dem Antikörper um einen monoklonalen Antikörper handelt.

24. Verfahren zur Überwachung des Expressionsniveaus des Krebs assoziierten Antigens nach Anspruch 1, wobei das Verfahren die Kontaktherstellung einer Probe mit einem Nukleinsäuremolekül nach Anspruch 2 und das Bestimmen der Hybridisierung der Nukleinsäure mit der genannten Probe umfasst.

25. Verfahren nach Anspruch 24, wobei das Verfahren die Verstärkung von RNA umfasst, die für das Krebs assoziierte Antigen nach Anspruch 1 codiert.

26. Verfahren nach Anspruch 25, wobei die Verstärkung eine Polymerase-Kettenreaktion umfasst.

27. Verfahren zur Überwachung des Wertes des Krebs assoziierten Antigens nach Anspruch 1, wobei das Verfahren die Kontaktherstellung einer Probe mit einem Antikörper nach Anspruch 22 oder Anspruch 23 umfasst sowie das Bestimmen der Bindung des Antikörpers an die Probe.

28. Verfahren nach Anspruch 27, wobei der Antikörper mit einem radioaktiven Label oder einem Enzym **gekennzeichnet** ist.

29. Verfahren nach Anspruch 27 oder Anspruch 28, wobei das Verfahren das Untersuchen der Probe auf ausgeschiedenes Protein umfasst.

30. Verfahren zum Testen einer Körperfluidprobe eines Patienten auf Immunzellenreaktivität auf eine eukaryotische Zelllinie nach einem der Ansprüche 4 bis 11, wobei das Verfahren die Kontaktherstellung einer eukaryotischen Zelle nach einem der Ansprüche 4 bis 11 mit der Probe umfasst.

31. Verfahren nach einem der Ansprüche 24 bis 30, wobei es sich bei der genannten Probe um ein Körperfluid oder Exsudate handelt.

32. Verfahren nach einem der Ansprüche 24 bis 30, wobei es sich bei der genannten Probe um ein Gewebe handelt.

## Revendications

1. Antigène isolé associé au cancer comprenant la séquence d'acides aminés codée par SEQ ID N° : 15 ; ou qui possède la séquence d'acides aminés de SEQ ID N° : 16 ou SEQ ID N° : 30.

2. Molécule d'acide nucléique isolée codant pour un antigène associé au cancer et comprenant la séquence nucléotidique de SEQ ID N° : 15, dans laquelle la molécule d'acide nucléique code pour un polypeptide qui possède la séquence d'acides aminés de SEQ ID N° : 16 ou SEQ ID N° : 30.

3. Vecteur d'expression comprenant une molécule d'acide nucléique isolée selon la revendication 2, lié de manière opérationnelle à un promoteur.

4. Lignée cellulaire eucaryote ou souche cellulaire procaryote, transformée ou transfectée avec un vecteur d'expression selon la revendication 3.

5. Lignée cellulaire eucaryote ou souche cellulaire procaryote selon la revendication 4, dans laquelle la lignée cellulaire est également transfectée avec une molécule d'acide nucléique codant pour une cytokine.

6. Lignée cellulaire eucaryote ou souche cellulaire procaryote selon la revendication 4 ou la revendication 5, dans laquelle ladite lignée cellulaire est par ailleurs transfectée par une molécule d'acide nucléique codant pour une molécule CMH.

7. Lignée cellulaire eucaryote ou souche cellulaire procaryote selon la revendication 5 ou la revendication 6, dans laquelle ladite cytokine est une interleukine.

8. Lignée cellulaire eucaryote ou souche cellulaire procaryote selon la revendication 7, dans laquelle l'interleukine est IL-2, IL-4 ou IL-12.

9. Lignée cellulaire eucaryote ou souche cellulaire procaryote selon l'une quelconque des revendications 5 à 8, dans laquelle la lignée cellulaire a été rendue non proliférative.

10. Lignée cellulaire eucaryote selon l'une quelconque des revendications 5 à 9, dans laquelle la lignée cellulaire est une lignée cellulaire de fibroblastes.

11. Lignée cellulaire eucaryote selon l'une quelconque des revendications 4 à 10 qui est une lignée cellulaire humaine.

12. Vecteur d'expression comprenant un virus muté ou atténué et une molécule d'acide nucléique isolée selon la revendication 2.

13. Vecteur d'expression selon la revendication 12, dans lequel ledit virus est un adénovirus ou le virus de la vaccine.

14. Vecteur d'expression selon la revendication 13, dans lequel le virus est le virus de la vaccine.

15. Vecteur d'expression selon la revendication 13, dans lequel le virus est un adénovirus.

16. Système d'expression utile dans la transfection d'une cellule, comprenant (i) un premier vecteur qui est un vecteur selon la revendication 3 et (ii) un second vecteur sélectionné parmi le groupe constitué de (a) un vecteur contenant une molécule d'acide nucléique qui code pour une molécule CMH ou HLA qui présente un antigène dérivé dudit antigène associé au cancer selon la revendication 1 et (b) un vecteur contenant une molécule d'acide nucléique qui code pour une interleukine.

17. Composition immunogène comprenant un antigène du cancer isolé selon la revendication 1 et un adjuvant pharmaceutiquement acceptable.

18. Composition immunogène comprenant au moins un peptide constitué d'une séquence d'acides aminés de 8 à 25 acides aminés ou de 8 à 12 acides aminés, concaténés les uns aux autres dans l'antigène du cancer isolé selon la revendication 1, et un adjuvant pharmaceutiquement acceptable.

19. Composition immunogène selon la revendication 17 ou la revendication 18, dans laquelle l'adjuvant est une cytokine, une saponine ou GM-CSF.

20. Composition immunogène selon la revendication 19, dans laquelle la composition comprend une pluralité de peptides qui forment un complexe avec une molécule CMH spécifique.

21. Vaccin pour traiter un sujet souffrant d'une affection cancéreuse comprenant une lignée cellulaire eucaryote selon l'une quelconque des revendications 4 à 11 et un adjuvant pharmacologiquement acceptable.

22. Anticorps isolé spécifique pour un antigène associé au cancer selon la revendication 1.

23. Anticorps isolé selon la revendication 22, dans lequel l'anticorps est un anticorps monoclonal.

24. Procédé pour surveiller le niveau d'expression de l'antigène associé au cancer selon la revendication 1, comprenant la mise en contact d'un échantillon avec une molécule d'acide nucléique selon la revendication 2 et la détermination de l'hybridation de l'acide nucléique avec ledit échantillon.

25. Procédé selon la revendication 24, comprenant l'amplification de l'ARN qui code pour l'antigène associé au cancer selon la revendication 1.

26. Procédé selon la revendication 25, dans lequel l'amplification comprend une réaction en chaîne par polymérase.

27. Procédé pour surveiller le niveau de l'antigène associé au cancer selon la revendication 1, comprenant la mise en contact d'un échantillon avec un anticorps selon la revendication 22 ou la revendication 23 et la détermination de la fixation de l'anticorps à l'échantillon.

28. Procédé selon la revendication 27, dans lequel l'anticorps est marqué avec un traceur radioactif ou une enzyme.

29. Procédé selon la revendication 27 ou la revendication 28, comprenant le dosage dudit échantillon pour de la protéine perdue.

30. Procédé de test d'un échantillon de fluide corporel d'un sujet pour la réactivité de ses cellules immunitaires à une lignée cellulaire eucaryote selon l'une quelconque des revendications 4 à 11, comprenant la mise en contact d'une cellule eucaryote selon l'une quelconque des revendications 4 à 11 avec l'échantillon.

31. Procédé selon l'une quelconque des revendications 24 à 30, dans lequel ledit échantillon est un fluide corporel ou des exsudats.

32. Procédé selon l'une quelconque des revendications 24 à 30, dans lequel ledit échantillon est un tissu.


<110> Jager, Dirk
   Scanlan, Matthew
   Gure, Ali
   Jager, Elke
   Knuth, Alexander
   Old, Lloyd
   Chen, Yao-tseng
<120> Isolated Nucleic Acid Molecules Encoding Cancer Associated Antigens, the Antigens per se, and Uses Thereof
<130> LUD 5615.2PCT
<140>
<141>
<150> US 09/451,739
<151> 2000-06-22
<150> US 09/451,739
<151> 1999-11-30
<160> 30
<210> 1
   <211> 1533
   <212> DNA
   <213> Homo sapiens
   <220>
   <221> CDS
   <222> 235
   <400> 1
   ggttttccac gttggacaag tgcggctcgg cggccagcgg agcgcgcccc ttcccgctgc   60
   ccgctccgct cctctcttct acccagccca gtgggcgagt gggcagcggc ggccgcggcg  120
   ctgggccctc tcccgccggt gtgtgcgcgc tcgtacgcgc ggcccccggc gccagccccg  180
   ccgcctgaga gggggcctgc gccgccggcc ggggcgtgcg cccgggagcc accgncaccg  240
   cggcccgcgc cctcaggcgc tggggtcccc gcggacccgg aggcggcgga cgggctcggc  300
   agatgtagcc gccgggccga agcaggagcc ggcggggggg cgccgggaga gcgagggctt  360
   tgcattttgc agtgctattt tttgaggggg gcggagggtg gaggaagtcg gaaagccgcg  420
   ccgagtcgcc ggggacctcc ggggtgaacc atgttgagtc ctgccaacgg ggagcagctc  480
   cacctggtga actatgtgga ggactacctg gactccatcg agtccctgcc tttcgacttg  540
   cagagaaatg tctcgctgat gcgggagatc gacgcgaaat accaagagat cctgaaggag  600
   ctagacgagt gctacgagcg cttcagtcgc gagacagacg gggcgcagaa gcggcggatg  660
   ctgcactgtg tgcagcgcgc gctgatccgc agccaggagc tgggcgacga gaagatccag  720
   atcgtgagcc agatggtgga gctggtggag aaccgcacgc ggcaggtgga cagccacgtg  780
   gagctgttcg aggcgcagca ggagctgggc gacacagcgg gcaacagcgg caaggctggc  840
   gcggacaggc ccaaaggcga ggcggcagcg caggctgaca agcccaacag caagcgctca  900
   cggcggcagc gcaacaacga gaaccgtgag aacgcgtcca gcaaccacga ccacgacgac  960
   ggcgcctcgg gcacacccaa ggagaagaag gccaagacct ccaagaagaa gaagcgctcc 1020
   aaggccaagg cggagcgaga ggcgtcccct gccgacctcc ccatcgaccc caacgaaccc 1080
   acgtactgtc tgtgcaacca ggtctcctat ggggagatga tcggctgcga caacgacgag 1140
   tgccccatcg agtggttcca cttctcgtgc gtggggctca atcataaacc caagggcaag 1200
   tggtactgtc ccaagtgccg gggggagaac gagaagacca tggacaaagc cctggagaaa 1260
   tccaaaaaag agagggctta caacaggtag tttgtggaca ggcgcctggt gtgaggagga 1320
   caaaataaac cgtgtattta ttacattgct gcctttgttg aggtgcaagg agtgtaaaat 1380
   gtatattttt aaagaatgtt agaaaaggaa ccattccttt catagggatg gcagtgattc 1440
   tgtttgcctt ttgttttcat tggtacacgt gtaacaagaa agtggtctgt ggatcagcat 1500
   tttagaaact acaaatatag gtttgattca aca                              1533
<210> 2
   <211> 1143
   <212> DNA
   <213> Homo sapiens
   <400> 2
   gagtaacccg ataatatgcc gttgtccggc acggcgacga gaattcccag atatagcagt 60
   agcagtgatc ccgggcctgt ggctcggggc cggggctgca gttcggaccg cctcccgcga 120
   cccgcggggg ctcggagaca gtttcaggcc gcatctttgc tgacccgagg gtggggccgc 180
   gcgtggccgt ggaaacagat cctgaaggag ctagacgagt gctacgagcg cttcagtcgc 240
   gagacagacg gggcgcagaa gcggcggatg ctgcactgtg tgcagcgcgc gctgatccgc 300
   agccaggagc tgggcgacga gaagatccag atcgtgagcc agatggtgga gctggtggag 360
   aaccgcacgc ggcaggtgga cagccacgtg gagctgttcg aggcgcagca ggagctgggc 420
   gacacagtgg gcaacagcgg caaggttggc gcggacaggc ccaatggcga tgcggtagcg 480
   cagtctgaca agcccaacag caagcgctca cggcggcagc gcaacaacga gaaccgtgag 540
   aacgcgtcca gcaaccacga ccacgacgac ggcgcctcgg gcacacccaa ggagaagaag 600
   gccaagacct ccaagaagaa gaagcgctcc aaggccaagg cggagcgaga ggcgtcccct 660
   gccgacctcc ccatcgaccc caacgaaccc acgtactgtc tgtgcaacca ggtctcctat 720
   ggggagatga tcggctgcga caacgacgag tgccccatcg agtggttcca cttctcgtgc 780
   gtggggctca atcataaacc caagggcaag tggtactgtc ccaagtgccg gggggagaac 840
   gagaagacca tggacaaagc cctggagaaa tccaaaaaag agagggctta caacaggtag 900
   tttgtggaca ggcgcctggt gtgaggagga caaaataaac cgtgtattta ttacattgct 960
   gcctttgttg aggtgcaagg agtgtaaaat gtatattttt aaagaatgtt agaaaaggaa 1020
   ccattccttt catagggatg gcagtgattc tgtttgcctt ttgttttcat tggtacacgt 1080
   gtaacaagaa agtggtctgt ggatcagcat tttagaaact acaaatatag gtttgattca 1140
   aca 1143
<210> 3
   <211> 742
   <212> DNA
   <213> Homo sapiens
<400> 3
   cgccgtccac accccagcgg ccctgacgct gtcccctccg cgaccctcgc ctctggaaaa 60
   agtgacaggc aaggccacgc cccgcgaggg gccggcctcg agcccgcagc ccccagggcc 120
   tgggacgaga tcctgaagga gctagacgag tgctacgagc gcttcagtcg cgagacagac  180
   ggggcgcaga agcggcggat gctgcactgt gtgcagcgcg cgctgatccg cagccaggag  240
   ctgggcgacg agaagatcca gatcgtgagc cagatggtgg agctggtgga gaaccgcacg  300
   cggcaggtgg acagccacgt ggagctgttc gaggcgcagc aggagctggg cgacacagcg  360
   ggcaacagcg gcaaggctgg cgcggacagg cccaaaggcg aggcggcagc gcaggctgac  420
   aagcccaaca gcaagcgctc acggcggcag cgcaacaacg agaaccgtga gaacgcgtcc  480
   agcaaccacg accacgacga cggcgcctcg ggcacaccca aggagaagaa ggccaagacc  540
   tccaagaaga agaagcgctc caaggccaag gcggagcgag aggcgtcccc tgccgacctc  600
   cccatcgacc ccaacgaacc cacgtactgt ctgtgcaacc aggtctccta tggggagatg  660
   atcggctgcg acaacgacga gtgccccatc gagtggttcc acttctcgtg cgtggggctc  720
   aatcataaac ccaagggcaa gt                                            742
<210> 4
   <211> 857
   <212> DNA
   <213> Homo sapiens
   <400> 4
   cctccgagaa cggtgtccat ggcacagggc gggaagagat aaggcctagg gaaggcgccc   60
   ctcgggccta tccacctctt ctggggctcg gcactaggaa gcagcttccc tctcaggccc  120
   ctttgtctcc aagccgttcc aaactgagta ccgggagacg acacaaaggg agggcggtga  180
   cggatggcgc aggcgcggga gccgcctagg ctgctgggag tggtggtccg gccgcggaat  240
   ggagatcctg aaggagctag acgagtgcta cgagcgcttc agtcgcgaga cagacggggc  300
   gcagaagcgg cggatgctgc actgtgtgca gcgcgcgctg atccgcagcc aggagctggg  360
   cgacgagaag atccagatcg tgagccagat ggtggagctg gtggagaacc gcacgcggca  420
   ggtggacagc cacgtggagc tgttcgaggc gcagcaggag ctgggcgaca cagcgggcaa  480
   cagcggcaag gctggcgcgg acaggcccaa aggcgaggcg gcagcgcagg ctgacaagcc  540
   caacagcaag cgctcacggc ggcagcgcaa caacgagaac cgtgagaacg cgtccagcaa  600
   ccacgaccac gacgacggcg cctcgggcac acccaaggag aagaaggcca agacctccaa  660
   gaagaagaag cgctccaagg ccaaggcgga gcgagaggcg tcccctgccg acctccccat  720
   cgaccccaac gaacccacgt actgtctgtg caaccaggtc tcctatgggg agatgatcgg  780
   ctgcgacaac gacgagtgcc ccatcgagtg gttccacttc tcgtgcgtgg ggctcaatca  840
   taaacccaag ggcaagt                                                  857
<210> 5
   <211> 279
   <212> PRT
   <213> Homo sapiens
   <400> 5
   Met Leu Ser Pro Ala Asn Gly Glu Gln Leu His Leu Val Asn Tyr Val
   1               5                   10                  15
   Glu Asp Tyr Leu Asp Ser Ile Glu Ser Leu Pro Phe Asp Leu Gln Arg
               20                  25                  30
   Asn Val Ser Leu Met Arg Glu Ile Asp Ala Lys Tyr Gln Glu Ile Leu
           35                  40                  45
   Lys Glu Leu Asp Glu Cys Tyr Glu Arg Phe Ser Arg Glu Thr Asp Gly
       50                  55                  60
   Ala Gln Lys Arg Arg Met Leu His Cys Val Gln Arg Ala Leu Ile Arg
   65                  70                  75                  80
   Ser Gln Glu Leu Gly Asp Glu Lys Ile Gln Ile Val Ser Gln Met Val
                   85                  90                  95
   Glu Leu Val Glu Asn Arg Thr Arg Gln Val Asp Ser His Val Glu Leu
               100                 105                 110
   Phe Glu Ala Gln Gln Glu Leu Gly Asp Thr Val Gly Asn Ser Gly Lys
           115                 120                 125
   Val Gly Ala Asp Arg Pro Asn Gly Asp Ala Val Ala Gln Ser Asp Lys
       130                 135                 140
   Pro Asn Ser Lys Arg Ser Arg Arg Gln Arg Asn Asn Glu Asn Arg Glu
   145                 150                 155                 160
   Asn Ala Ser Ser Asn His Asp His Asp Asp Gly Ala Ser Gly Thr Pro
                   165                 170                 175
   Lys Glu Lys Lys Ala Lys Thr Ser Lys Lys Lys Lys Arg Ser Lys Ala
               180                 185                 190
   Lys Ala Glu Arg Glu Ala Ser Pro Ala Asp Leu Pro Ile Asp Pro Asn
           195                 200                 205
   Glu Pro Thr Tyr Cys Leu Cys Asn Gln Val Ser Tyr Gly Glu Met Ile
       210                 215                 220
   Gly Cys Asp Asn Asp Glu Cys Pro Ile Glu Trp Phe His Phe Ser Cys
   225                 230                 235                 240
   Val Gly Leu Asn His Lys Pro Lys Gly Lys Trp Tyr Cys Pro Lys Cys
                   245                 250                 255
   Arg Gly Glu Asn Glu Lys Thr Met Asp Lys Ala Leu Glu Lys Ser Lys
               260                 265                 270
   Lys Glu Arg Ala Tyr Asn Arg
           275
<210> 6
   <211> 210
   <212> PRT
   <213> Homo sapiens
   <400> 6
   Met Leu His Cys Val Gln Arg Ala Leu Ile Arg Ser Gln Glu Leu Gly
   1 5 10 15
   Asp Glu Lys Ile Gln Ile Val Ser Gln Met Val Glu Leu Val Glu Asn
   20 25 30
   Arg Thr Arg Gln Val Asp Ser His Val Glu Leu Phe Glu Ala Gln Gln
   35 40 45
   Glu Leu Gly Asp Thr Val Gly Asn Ser Gly Lys Val Gly Ala Asp Arg
   50 55 60
   Pro Asn Gly Asp Ala Val Ala Gln Ser Asp Lys Pro Asn Ser Lys Arg
   65 70 75 80
   Ser Arg Arg Gln Arg Asn Asn Glu Asn Arg Glu Asn Ala Ser Ser Asn
   85 90 95
   His Asp His Asp Asp Gly Ala Ser Gly Thr Pro Lys Glu Lys Lys Ala
   100 105 110
   Lys Thr Ser Lys Lys Lys Lys Arg Ser Lys Ala Lys Ala Glu Arg Glu
   115 120 125
   Ala Ser Pro Ala Asp Leu Pro Ile Asp Pro Asn Glu Pro Thr Tyr Cys
   130 135 140
   Leu Cys Asn Gln Val Ser Tyr Gly Glu Met Ile Gly Cys Asp Asn Asp
   145 150 155 160
   Glu Cys Pro Ile Glu Trp Phe His Phe Ser Cys Val Gly Leu Asn His
   165 170 175
   Lys Pro Lys Gly Lys Trp Tyr Cys Pro Lys Cys Arg Gly Glu Asn Glu
   180 185 190
   Lys Thr Met Asp Lys Ala Leu Glu Lys Ser Lys Lys Glu Arg Ala Tyr
   195 200 205
   Asn Arg
   210
<210> 7
   <211> 235
   <212> PRT
   <213> Homo sapiens
   <400> 7
<210> 8
   <211> 772
   <212> DNA
   <213> Homo sapiens
   <221> CDS
   <222> 695,714
   <400> 8
   aaagcgttct cggcggcagc gcaacaacta gaaccgtgag aacgcgtcca gcaaccgcga   60
   cccacgacga cgtcacctcg ggcacgccca aggagaagaa agcccagacc tctaagaaga  120
   agcagggctc catggccaag gcgtagcggc aggcgtcccc cgcagacctc cccatcgacc  180
   ccagcgagcc ctcctactgg gagatgatcc gctgcgacaa cgaatgcccc atcgagtggt  240
   tccgcttctc gtgtgtgagt ctcaaccata aaccaaagcg caagtggtac tgttccagat  300
   gccggggaaa gaacgatggg caaagccctt gagaagtcca gaaaaaaaac agggcttata  360
   acaggtagtt tggggacatg cgtctaatag tgaggagaac aaaataagcc agtgtgttga  420
   ttacattgcc acctttgctg aggtgcagga agtgtaaaat gtatattttt aaagaatgtt  480
   gttagaggcc gggcgcggtg gctcacgcct gtaatcccag cactttggga ggccgaggcg  540
   gtcggatcac gaggtcagga gatcgagacc atcctggcta acacggtgaa accccgtctc  600
   tactaaaaat tcaaaaaaaa aattagctgg gcgtggtggc gggcgcctgt agtcccagct  660
   attcgggagg ctgaggcagg agaatggcnt gaacctggga ggtggagctt gcantgagcc  720
   aaggtcgcgc cactgcactc cagcctgggc gacagagcga gactccatct ta          772
<210> 9
   <211> 32
   <212> DNA
   <213> Homo sapiens
   <400> 9
   cacacaggat ccatgttgag tcctgccaac gg 32
<210> 10
   <211> 23
   <212> DNA
   <213> Homo sapiens
<400> 10
   cgtggtcgtg gttgctggac gcg 23
<210> 11
   <211> 21
   <212> DNA
   <213> Homo sapiens
   <400> 11
   cccagcggcc ctgacgctgt c 21
<210> 12
   <211> 23
   <212> DNA
   <213> Homo sapiens
   <400> 12
   cgtggtcgtg gttgctggac gcg 23
<210> 13
   <211> 23
   <212> DNA
   <213> Homo sapiens
   <400> 13
   ggaagagata aggcctaggg aag 23
<210> 14
   <211> 23
   <212> DNA
   <213> Homo sapiens
   <400> 14
   cgtggtcgtg gttgctggac gcg 23
<210> 15
   <211> 2030
   <212> DNA
   <213> Homo sapiens
   <221> CDS
   <222> 1628, 1752, 1758, 1769, 1789, 1873, 1908, 1915, 1933, 1970, 1976, 2022 <400> 15
<210> 16
   <211> 513
   <212> PRT
   <213> Homo sapiens
   <400> 16
<210> 17
   <211> 33
   <212> DNA
   <213> Homo sapiens
   <400> 17
   cacacaggat ccatgcaggc cccgcacaag gag 33
<210> 18
   <211> 34
   <212> DNA
   <213> Homo sapiens
   <400> 18
   cacacaaagc ttctaggatt tggcacagcc agag 34
<210> 19
   <211> 294
   <212> PRT
   <213> Homo sapiens
   <400> 19
   Met Pro Leu Cys Thr Ala Thr Arg Ile Pro Arg Tyr Ser Ser Ser Ser
   1               5                   10                  15
   Asp Pro Gly Pro Val Ala Arg Gly Arg Gly Cys Ser Ser Asp Arg Leu
               20                  25                  30
   Pro Arg Pro Ala Gly Pro Ala Arg Arg Gln Phe Gln Ala Ala Ser Leu
           35                  40                  45
   Leu Thr Arg Gly Trp Gly Arg Ala Trp Pro Trp Lys Gln Ile Leu Lys
       50                  55                  60
   Glu Leu Asp Glu Cys Tyr Glu Arg Phe Ser Arg Glu Thr Asp Gly Ala
   65                  70                  75                  80
   Gln Lys Arg Arg Met Leu His Cys Val Gln Arg Ala Leu Ile Arg Ser
                   85                  90                  95
   Gln Glu Leu Gly Asp Glu Lys Ile Gln Ile Val Ser Gln Met Val Glu
               100                 105                 110
   Leu Val Glu Asn Arg Thr Arg Gln Val Asp Ser His Val Glu Leu Phe
           115                 120                 125
   Glu Ala Gln Gln Glu Leu Gly Asp Thr Val Gly Asn Ser Gly Lys Val
       130                 135                 140
   Gly Ala Asp Arg Pro Asn Gly Asp Ala Val Ala Gln Ser Asp Lys Pro
   145                 150                 155                 160
   Asn Ser Lys Arg Ser Arg Arg Gln Arg Asn Asn Glu Asn Arg Glu Asn
                   165                 170                 175
   Ala Ser Ser Asn His Asp His Asp Asp Gly Ala Ser Gly Thr Pro Lys
               180                 185                 190
   Glu Lys Lys Ala Lys Thr Ser Lys Lys Lys Lys Arg Ser Lys Ala Lys
           195                 200                 205
   Ala Glu Arg Glu Ala Ser Pro Ala Asp Leu Pro Ile Asp Pro Asn Glu
       210                 215                 220
   Pro Thr Tyr Cys Leu Cys Asn Gln Val Ser Tyr Gly Glu Met Ile Gly
   225                 230                 235                 240
   Cys Asp Asn Asp Glu Cys Pro Ile Glu Trp Phe His Phe Ser Cys Val
                   245                 250                 255
   Gly Leu Asn His Lys Pro Lys Gly Lys Trp Tyr Cys Pro Lys Cys Arg
               260                 265                 270
   Gly Glu Asn Glu Lys Thr Met Asp Lys Ala Leu Glu Lys Ser Lys Lys
           275                 280                 285
   Glu Arg Ala Tyr Asn Arg
       290             294
<210> 20
   <211> 22
   <212> DNA
   <213> Homo sapiens
   <400> 20
   caaagcagag cctcccgaga ag 22
<210> 21
   <211> 24
   <212> DNA
   <213> Homo sapiens
   <400> 21
   cctatgctgc tcttcgattc ttcc 24
<210> 22
   <211> 4115
   <212> DNA
   <213> Homo sapiens
   <400> 22
   ctagtctata cagcaacgac cctacatcgt cactctgggg tcttagaaag tccataaagc   60
   tgcctcccgg gacaagtccg aagctggaga gatgacaaag ggaagaagac atcaacctta  120
   atatacaaga gcccagaaga gactgctcta actgggcctg gtcaatggcc tgaggaagta  180
   gtaacatttc ggtagacaga agtgccagct gacgtccttg tggcgaacac ggacacctct  240
   gatgaaggct tacaatgcca caggaggctt tgcaaatatt tgatagattc ggtgccgata  300
   taaatctcgt gatgtgtatg caacatggct tccattatgc gtttatagtg gattttgtca  360
   gtggtggcaa actgctgtcc atggtgcagt atcgaagtgc caacaaggct gcctcacacc  420
   acttttacta ccataacgaa agaagtgagc aattgtggaa ttttgctgat aaaaatgcaa  480
   atgcgaatgc gttaataagt taaatgcaca ccctcatgct gctgtatgtc tggatcatca  540
   gagatagttg catgcttctt agcaaaatgt gacgtctttg tgcagatata gtggagtaac  600
   tgcagaacat atgctgttac tgtggatttc tcacattcat aacaaattat gaatatatac  660
   gaaaattatc aaaaatcatc aaataccaat cagaaggaac tctgcaggaa acctgatgag  720
   gctgcaccct ggcggaaaga cacctgacac gctgaaagct ggtggaaaaa cacctgatga  780
   ggctgcaccc tggtggaaag acacctgaca ggctgaaagc tggtggaaaa acacctgatg  840
   aggctgcatc ttggtggagg aacatctgac aaattcaatg ttggagaaag gacatctgga  900
   aagttcgaac gtcagcagaa aaacacctag gaaattacga tcctgcaaaa aaacatctga  960
   gaaatttacg ggccagcaaa ggaagaccta gaagatcgca gggagaaaaa gaagacacac 1020
   ctagggaaat atgagtcccg aaaagaaaca ctgagaaatt acgtgggcag aaaaggaaga 1080
   cctaggaaga cgcatgggag aaaaagaaac cctgtaaaga tggatgcgtg caagagtaac 1140
   atctaataaa ctaaagtttt gaaaaaggaa atctaagatg ttgcatgtcc acaaaagaat 1200
   catctacaaa gcaagtgcca tgatcagagg tcccatcaga tccaaacaag ggaagatgaa 1260
   gaatattctt tgattctcgg gtctctttga agttctgcaa gattcaagtg gtatacctga 1320
   gtctatatat aaaaagtaat gagataaata agaagtagaa agcctcctaa aagccatctg 1380
   ccttcaagcc gccattgaaa gcaaaactct ttccaaataa gcctttgaat gaagaatgaa 1440
   caaacattga agcagatccg tgttcccacc gaatccaaac aaaggactat aagaaaattc 1500
   ttgggattct agagtctctg gagactgttt acagaaggat tgtgtttacc aaggctacac 1560
   atcaaaaaga atagataaaa aaatggaaaa tagaagagtc cctaataaag tggtcttctg 1620
   aaggctacct cggaatgaaa tttctattcc actaaagcct agaattgaag acatgcaaac 1680
   tttcaaagcg agcctccggg aagccatctg cttcgagcct ccactgaaat caaaagtctg 1740
   tcccaaataa gccttggaat gaaaaatgaa aaacatggag gcagatgaga actcccatca 1800
   gaatccaaac aaaggactat aagaaaattc tgggatactg gagtctctgt agactgtttc 1860
   acagaaggat tgtgtttacc aaggctgcgc tcaaaaagaa tagataaaat aatggaaaat 1920
   tagaagggtc cctgttaaag tggtcttctg aggctaactg ggaatgaaag ttctattcca 1980
   actaaagcct agaattgatg acatgcaaac ttcaaagcag gcctcccgag agccatctgc 2040
   cttcgagcct ccattgaaat caaaagtctg tccaaataaa ccttggaatt aagaatgaac 2100
   aaacattgag gcagatgaga actcccatca aatccaaaca aaggactatg agaaagttct 2160
   tgggattctg gagtctctgt agactgtttc cagaaggatg gtgtttaccc aggctacaca 2220
   tcaaaaagaa tagataaaat aatggaaaat agaagagtct ctgataatga ggttttctga 2280
   aggctccctg agaatgaaag ttctattcca ctaaagcctt gaattgatgg catgcaaact 2340
   ttcaaagcag gcctcccgag agccatctgc ttcgagcctg cattgaaatg aaaagtctgt 2400
   tccaaataaa ccttggaatt aagaatgaac aacattgaga cagatcagat ttcccttcag 2460
   aatcaaaaca aagaaggttg agaaaattct gggattctga agtctccgtg gactgtttca 2520
   cagaaggatg gtgtgtaccc aggctacaca caaaaagaaa ggataaaata gtggaaaatt 2580
   agaagattca ctagcctatc aaaatcttgg tacagttcat cttgtgaaag gcaagggaac 2640
   ttcaaaaaga cactgtgaac acgtacagga aaatggaaca atgaaaaaga gttttgtgta 2700
   ctgaaaaaga actgtcagaa caaaagaaat aaatcacagt agagaaccaa aagttaaatg 2760
   ggaacaagag tctgcagtgt agattgactt aaaccaagaa aagagaagag agaaatgccg 2820
   atatattaaa gaaaaaatta ggaagaatta gaagaatcga gagcagcata gaaagagtta 2880
   gaagtgaaac acaacttgaa aggctctcag atacaagata agaattgaag gtgtagaaag 2940
   taatttgaat aggtttctca actcatgaaa tgaaaattat tcttacatga aattgcatgt 3000
   tgaaaaagga attgccatgc aaaactggaa tagccacact aaacaccaat ccaggaaaag 3060
   gaaaataaat ctttgaggac ttaagatttt aaagaaaaga tgctgaactt agatgaccct 3120
   aaaactgaaa aggaatcatt actaaaaggg atctcaatat gtgggcagct aaagttctga 3180
   tagctgagaa acaatgctca ttctaaattg aggaaaaaca gacaaagaaa actagaggca 3240
   gaaattgaat acaccatcct gactggcttc gctgtacaag ccatgatcaa ttgtgacatc 3300
   aagaaaaagt aagaacctgc ttccacattg aggagatgct gtttgcaaag aaaatgaatg 3360
   ttgatgtgag agtacgatat taacaatgag tgctccatca ccactttctg agctcaaagg 3420
   aaatccaaaa cctaaaaatt atctcaatta gcaggagatg tctaagagaa atacattggt 3480
   ttcagaacat cacaaagaga caacgtgaaa acagtgtcaa tgaaggaagc gaacacatgt 3540
   atcaaaacga caagataatg gaacaaacac ctgaacagca gagtctctag tcagaaatta 3600
   tttcaactac aagcaaaaat tgtggcttca cagcaattag tcatgcacat agaaagctga 3660
   caacaaaagc agataacaat gatattcatt tcttgagagg aaatgcaaca catctcctaa 3720
   aagagaaaaa gaggagatat taattacaat accatttaaa aaccgtatat tcaatatgaa 3780
   aaagagaaag agaaacagaa actcatgaga acaagcagta gaaacttctt tggagaaaca 3840
   acagaccaga ctttactcac actcatgcta gaggccagtc tagcatcacc tatgttgaaa 3900
   atcttaccaa agtctgtgtc acagaatact attttagaag aaaattcatg tttcttcctg 3960
   aagcctacag cataaaataa agtgtgaaga ttacttgttc cgaattgcat aagctgcaca 4020
   ggattcccat taccctgatg tgcagcagac tcattcaatc aaccagaatc cgctctgcac 4080
   tccagcctag tgacagagtg gactccacct ggaaa 4115
<210> 23
   <211> 1341
   <212> PRT
   <213> Homo sapiens
   <400> 23
   Met Thr Lys Arg Lys Lys Thr Ile Asn Leu Asn Ile Gln Asp Ala Gln
   1               5                   10                  15
   Lys Arg Thr Ala Leu His Trp Ala Cys Val Asn Gly His Glu Glu Val
               20                  25                  30
   Val Thr Phe Leu Val Asp Arg Lys Cys Gln Leu Asp Val Leu Asp Gly
           35                  40                  45
   Glu His Arg Thr Pro Leu Met Lys Ala Leu Gln Cys His Gln Glu Ala
       50                  55                  60
   Cys Ala Asn Ile Leu Ile Asp Ser Gly Ala Asp Ile Asn Leu Val Asp
   65                  70                  75                  80
   Val Tyr Gly Asn Met Ala Leu His Tyr Ala Val Tyr Ser Glu Ile Leu
                   85                  90                  95
   Ser Val Val Ala Lys Leu Leu Ser His Gly Ala Val Ile Glu Val His
               100                 105                 110
   Asn Lys Ala Ser Leu Thr Pro Leu Leu Leu Ser Ile Thr Lys Arg Ser
           115                 120                 125
   Glu Gln Ile Val Glu Phe Leu Leu Ile Lys Asn Ala Asn Ala Asn Ala
       130                 135                 140
   Val Asn Lys Tyr Lys Cys Thr Ala Leu Met Leu Ala Val Cys His Gly
   145                 150                 155                 160
   Ser Ser Glu Ile Val Gly Met Leu Leu Gln Gln Asn Val Asp Val Phe
                   165                 170                 175
   Ala Ala Asp Ile Cys Gly Val Thr Ala Glu His Tyr Ala Val Thr Cys
               180                 185                 190
   Gly Phe His His Ile His Glu Gln Ile Met Glu Tyr Ile Arg Lys Leu
           195                 200                 205
   Ser Lys Asn His Gln Asn Thr Asn Pro Glu Gly Thr Ser Ala Gly Thr
       210                 215                 220
   Pro Asp Glu Ala Ala Pro Leu Ala Glu Arg Thr Pro Asp Thr Ala Glu
   225                 230                 235                 240
   Ser Leu Val Glu Lys Thr Pro Asp Glu Ala Ala Pro Leu Val Glu Arg
                   245                 250                 255
   Thr Pro Asp Thr Ala Glu Ser Leu Val Glu Lys Thr Pro Asp Glu Ala
               260                 265                 270
   Ala Ser Leu Val Glu Gly Thr Ser Asp Lys Ile Gln Cys Leu Glu Lys
           275                 280                 285
   Ala Thr Ser Gly Lys Phe Glu Gln Ser Ala Glu Glu Thr Pro Arg Glu
       290                 295                 300
   Ile Thr Ser Pro Ala Lys Glu Thr Ser Glu Lys Phe Thr Trp Pro Ala
   305                 310                 315                 320
   Lys Gly Arg Pro Arg Lys Ile Ala Trp Glu Lys Lys Glu Asp Thr Pro
                   325                 330                 335
   Arg Glu Ile Met Ser Pro Ala Lys Glu Thr Ser Glu Lys Phe Thr Trp
               340                 345                 350
   Ala Ala Lys Gly Arg Pro Arg Lys Ile Ala Trp Glu Lys Lys Glu Thr
           355                 360                 365
   Pro Val Lys Thr Gly Cys Val Ala Arg Val Thr Ser Asn Lys Thr Lys
       370                 375                 380
   Val Leu Glu Lys Gly Arg Ser Lys Met Ile Ala Cys Pro Thr Lys Glu
   385 390 395 400
   Ser Ser Thr Lys Ala Ser Ala Asn Asp Gln Arg Phe Pro Ser Glu Ser
   405 410 415
   Lys Gln Glu Glu Asp Glu Glu Tyr Ser Cys Asp Ser Arg Ser Leu Phe
   420 425 430
   Glu Ser Ser Ala Lys Ile Gln Val Cys Ile Pro Glu Ser Ile Tyr Gln
   435 440 445
   Lys Val Met Glu Ile Asn Arg Glu Val Glu Glu Pro Pro Lys Lys Pro
   450 455 460
   Ser Ala Phe Lys Pro Ala Ile Glu Met Gln Asn Ser Val Pro Asn Lys
   465 470 475 480
   Ala Phe Glu Leu Lys Asn Glu Gln Thr Leu Arg Ala Asp Pro Met Phe
   485 490 495
   Pro Pro Glu Ser Lys Gln Lys Asp Tyr Glu Glu Asn Ser Trp Asp Ser
   500 505 510
   Glu Ser Leu Cys Glu Thr Val Ser Gln Lys Asp Val Cys Leu Pro Lys
   515 520 525
   Ala Thr His Gln Lys Glu Ile Asp Lys Ile Asn Gly Lys Leu Glu Glu
   530 535 540
   Ser Pro Asn Lys Asp Gly Leu Leu Lys Ala Thr Cys Gly Met Lys Val
   545 550 555 560
   Ser Ile Pro Thr Lys Ala Leu Glu Leu Lys Asp Met Gln Thr Phe Lys
   565 570 575
   Ala Glu Pro Pro Gly Lys Pro Ser Ala Phe Glu Pro Ala Thr Glu Met
   580 585 590
   Gln Lys Ser Val Pro Asn Lys Ala Leu Glu Leu Lys Asn Glu Gln Thr
   595 600 605
   Trp Arg Ala Asp Glu Ile Leu Pro Ser Glu Ser Lys Gln Lys Asp Tyr
   610 615 620
   Glu Glu Asn Ser Trp Asp Thr Glu Ser Leu Cys Glu Thr Val Ser Gln
   625 630 635 640
   Lys Asp Val Cys Leu Pro Lys Ala Ala His Gln Lys Glu Ile Asp Lys
   645 650 655
   Ile Asn Gly Lys Leu Glu Gly Ser Pro Val Lys Asp Gly Leu Leu Lys
   660 665 670
   Ala Asn Cys Gly Met Lys Val Ser Ile Pro Thr Lys Ala Leu Glu Leu
   675 680 685
   Met Asp Met Gln Thr Phe Lys Ala Glu Pro Pro Glu Lys Pro Ser Ala
   690 695 700
   Phe Glu Pro Ala Ile Glu Met Gln Lys Ser Val Pro Asn Lys Ala Leu
   705 710 715 720
   Glu Leu Lys Asn Glu Gln Thr Leu Arg Ala Asp Glu Ile Leu Pro Ser
   725 730 735
   Glu Ser Lys Gln Lys Asp Tyr Glu Glu Ser Ser Trp Asp Ser Glu Ser
   740 745 750
   Leu Cys Glu Thr Val Ser Gln Lys Asp Val Cys Leu Pro Lys Ala Thr
   755 760 765
   His Gln Lys Glu Ile Asp Lys Ile Asn Gly Lys Leu Glu Glu Ser Pro
   770 775 780
   Asp Asn Asp Gly Phe Leu Lys Ala Pro Cys Arg Met Lys Val Ser Ile
   785 790 795 800
   Pro Thr Lys Ala Leu Glu Leu Met Asp Met Gln Thr Phe Lys Ala Glu
   805 810 815
   Pro Pro Glu Lys Pro Ser Ala Phe Glu Pro Ala Ile Glu Met Gln Lys
   820 825 830
   Ser Val Pro Asn Lys Ala Leu Glu Leu Lys Asn Glu Gln Thr Leu Arg
   835 840 845
   Ala Asp Gln Met Phe Pro Ser Glu Ser Lys Gln Lys Lys Val Glu Glu
   850 855 860
   Asn Ser Trp Asp Ser Glu Ser Leu Arg Glu Thr Val Ser Gln Lys Asp
   865 870 875 880
   Val Cys Val Pro Lys Ala Thr His Gln Lys Glu Met Asp Lys Ile Ser
   885 890 895
   Gly Lys Leu Glu Asp Ser Thr Ser Leu Ser Lys Ile Leu Asp Thr Val
   900 905 910
   His Ser Cys Glu Arg Ala Arg Glu Leu Gln Lys Asp His Cys Glu Gln
   915 920 925
   Arg Thr Gly Lys Met Glu Gln Met Lys Lys Lys Phe Cys Val Leu Lys
   930 935 940
   Lys Lys Leu Ser Glu Ala Lys Glu Ile Lys Ser Gln Leu Glu Asn Gln
   945 950 955 960
   Lys Val Lys Trp Glu Gln Glu Leu Cys Ser Val Arg Leu Thr Leu Asn
   965 970 975
   Gln Glu Glu Glu Lys Arg Arg Asn Ala Asp Ile Leu Asn Glu Lys Ile
   980 985 990
   Arg Glu Glu Leu Gly Arg Ile Glu Glu Gln His Arg Lys Glu Leu Glu
   995 1000 1005
   Val Lys Gln Gln Leu Glu Gln Ala Leu Arg Ile Gln Asp Ile Glu Leu
   1010 1015 1020
   Lys Ser Val Glu Ser Asn Leu Asn Gln Val Ser His Thr His Glu Asn
   1025 1030 1035 1040
   Glu Asn Tyr Leu Leu His Glu Asn Cys Met Leu Lys Lys Glu Ile Ala
   1045 1050 1055
   Met Leu Lys Leu Glu Ile Ala Thr Leu Lys His Gln Tyr Gln Glu Lys
   1060 1065 1070
   Glu Asn Lys Tyr Phe Glu Asp Ile Lys Ile Leu Lys Glu Lys Asn Ala
   1075 1080 1085
   Glu Leu Gln Met Thr Leu Lys Leu Lys Glu Glu Ser Leu Thr Lys Arg
   1090 1095 1100
   Ala Ser Gln Tyr Ser Gly Gln Leu Lys Val Leu Ile Ala Glu Asn Thr
   1105 1110 1115 1120
   Met Leu Thr Ser Lys Leu Lys Glu Lys Gln Asp Lys Glu Ile Leu Glu
   1125 1130 1135
   Ala Glu Ile Glu Ser His His Pro Arg Leu Ala Ser Ala Val Gln Asp
   1140 1145 1150
   His Asp Gln Ile Val Thr Ser Arg Lys Ser Gln Glu Pro Ala Phe His
               1155                1160                1165
   Ile Ala Gly Asp Ala Cys Leu Gln Arg Lys Met Asn Val Asp Val Ser
       1170                1175                1180
   Ser Thr Ile Tyr Asn Asn Glu Val Leu His Gln Pro Leu Ser Glu Ala
   1185                1190                1195                1200
   Gln Arg Lys Ser Lys Ser Leu Lys Ile Asn Leu Asn Tyr Ala Gly Asp
                   1205                1210                1215
   Ala Leu Arg Glu Asn Thr Leu Val Ser Glu His Ala Gln Arg Asp Gln
               1220                1225                1230
   Arg Glu Thr Gln Cys Gln Met Lys Glu Ala Glu His Met Tyr Gln Asn
           1235                1240                1245
   Glu Gln Asp Asn Val Asn Lys His Thr Glu Gln Gln Glu Ser Leu Asp
       1250                1255                1260
   Gln Lys Leu Phe Gln Leu Gln Ser Lys Asn Met Trp Leu Gln Gln Gln
   1265                1270                1275                1280
   Leu Val His Ala His Lys Lys Ala Asp Asn Lys Ser Lys Ile Thr Ile
                   1285                1290                1295
   Asp Ile His Phe Leu Glu Arg Lys Met Gln His His Leu Leu Lys Glu
               1300                1305                1310
   Lys Asn Glu Glu Ile Phe Asn Tyr Asn Asn His Leu Lys Asn Arg Ile
           1315                1320                1325
   Tyr Gln Tyr Glu Lys Glu Lys Ala Glu Thr Glu Asn Ser
       1330                1335                1340
<210> 24
   <211> 22
   <212> DNA
   <213> Homo sapiens
   <400> 24
   aatgggaaca agagctctgc ag 22
<210> 25
   <211> 23
   <212> DNA
   <213> Homo sapiens
   <400> 25
   gggtcatctg aagttcagca ttc 23
<210> 26
   <211> 3673
   <212> DNA
   <213> Homo sapiens
   <221> CDS
   <222> 439, 473, 1789
   <400> 26
   caagagcttg gcgatacaga aatttctgct ggtgttgggg cgggtgcggg aactgaagac   60
   gggcgagtgc gagccggggg cgggtgctgg ggaagggtaa gcgggaagcg agggcgaggg  120
   gtaggggctg gggaagggcg agcgggaggc gcgggctctc tctagcaggg ggctgcagcc  180
   atgaagaggc tcttagctgc cgctggcaag ggcgtgcggg gcccggagcc cccgaacccc  240
   ttcagcgaac gggtctacac tgagaaggac tacgggacca tctacttcgg ggatctaggg  300
   aagatccata cagctgcctc ccggggccaa gtccagaagc tggagaagat gacagtaggg  360
   aagaagcccg tcaacctgaa caaaagagat atgaagaaga ggactgctct acactgggcc  420
   tgtgtcaatg gccatgcana agtagtaaca tttctggtag acagaaagtg ccngcttaat 480
   gtccttgatg gcgaagggag gacacctctg atgaaggctc tacaatgcga gagggaagct 540
   ttgtgcaaat attctcatag atgctggtgc tgatctaaat tatgtagatg tgtatggcaa 600
   cacggctctc cattatgccg tttatagtga gaatttatta atggtggcaa cactgctgtc 660
   ctatggtgca gtcatcgagg tgcaaaacaa ggctagcctc acaccccttt tactggccat 720
   acagaaaaga agcaagcaaa ctgtggaatt tttactaaca aaaaatgcaa atgcaaacgc 780
   atttaatgag tctaaatgca cagccctcat gcttgccata tgtgaaggct catcagagat 840
   agtcggcatg cttcttcagc aaaatgttga cgtctttgct gaagacatac atggaataac 900
   tgcagaacgt tatgctgctg ctcgtggagt taattacatt catcaacaac ttttggaaca 960
   tatacgaaaa ttacctaaaa atcctcaaaa taccaatcca gaaggaacat ctacaggaac 1020
   acctgatgag gctgcaccct tggcggaaag aacacctgac acggctgaaa gcttgctgga 1080
   aaaaacacct gacgaggctg cacgcttggt ggagggaacg tctgccaaaa ttcaatgtct 1140
   ggggaaagca acatctggaa agtttgaaca gtcaacagaa gaaacaccta ggaaaatttt 1200
   gaggcctaca aaagaaacat ctgagaaatt ttcatggcca gcaaaagaaa gatctaggaa 1260
   gatcacatgg gaggaaaaag aaacatctgt aaagactgaa tgcgtggcag gagtaacacc 1320
   taataaaact gaagttttgg aaaaaggaac atctaatatg attgcatgtc ctacaaaaga 1380
   aacatctaca aaagcaagta caaatgtgga tgtgagttct gtagagccta tattcagtct 1440
   ttttggcaca cggactattg aaaattcaca gtgtacaaaa gttgaggaag actttaatct 1500
   tgctaccaag attatctcta agagtgctgc acagaattat acgtgtttac ctgatgctac 1560
   atatcaaaaa gatatcaaaa caataaatca caaaatagaa gatcagatgt tcccatcaga 1620
   atccaaacga gaggaagatg aagaatattc ttgggattct gggagtctct ttgagagttc 1680
   tgcaaagact caagtgtgta tacctgagtc tatgtatcag aaagtaatgg agataaatag 1740
   agaagtagaa gagcttcctg agaagccatc tgccttcaag cctgccgtng aaatgcaaaa 1800
   gactgttcca aataaagcct ttgaattgaa gaatgaacaa acattgagag cagctcagat 1860
   gttcccatca gaatccaaac aaaaggacga tgaagaaaat tcttgggatt ctgagagtcc 1920
   ctgtgagacg gtttcacaga aggatgtgta tttacccaaa gctacacatc aaaaagaatt 1980
   cgatacctta agtggaaaat tagaagagtc tcctgttaaa gatggtcttc tgaagcctac 2040
   ctgtggaagg aaagtttctc ttccaaataa agccttagaa ttaaaggaca gagaaacatt 2100
   caaagcagag tctcctgata aagatggtct tctgaagcct acctgtggaa ggaaagtttc 2160
   tcttccaaat aaagccttag aattaaagga cagagaaaca ctcaaagcag agtctcctga 2220
   taatgatggt cttctgaagc ctacctgtgg aaggaaagtt tctcttccaa ataaagcttt 2280
   agaattgaag gacagagaaa cattcaaagc agctcagatg ttcccatcag aatccaaaca 2340
   aaaggatgat gaagaaaatt cttgggattt tgagagtttc cttgagactc tcttacagaa 2400
   tgatgtgtgt ttacccaagg ctacacatca aaaagaattc gataccttaa gtggaaaatt 2460
   agaagagtct cctgataaag atggtcttct gaagcctacc tgtggaatga aaatttctct 2520
   tccaaataaa gccttagaat tgaaggacag agaaacattc aaagcagagg atgtgagttc 2580
   tgtagagtcc acattcagtc tttttggcaa accgactact gaaaattcac agtctacaaa 2640
   agttgaggaa gactttaatc ttactaccaa ggagggagca acaaagacag taactggaca 2700
   acaggaacgt gatattggca ttattgaacg agctccacaa gatcaaacaa ataagatgcc 2760
   cacatcagaa ttaggaagaa aagaagatac aaaatcaact tcagattctg agattatctc 2820
   tgtgagtgat acacagaatt atgagtgttt acctgaggct acatatcaaa aagaaataaa 2880
   gacaacaaat ggcaaaatag aagagtctcc tgaaaagcct tctcactttg agcctgccac 2940
   tgaaatgcaa aactctgttc caaataaagg cttagaatgg aagaataaac aaacattgag 3000
   agcagattca actaccctat caaaaatctt ggatgcactt ccttcttgtg aaagaggaag 3060
   ggaacttaaa aaagataact gtgaacaaat tacagcaaaa atggaacaaa tgaaaaataa 3120
   gttttgtgta ctacaaaagg aactgtcaga agcgaaagaa ataaaatcac agttagagaa 3180
   ccaaaaagct aaatgggaac aagagctctg cagtgtgaga ttgcctttaa atcaagaaga 3240
   agagaagaga agaaatgtcg atatattaaa agaaaaaatt agacccgaag agcaacttag 3300
   gaaaaagtta gaagtgaaac accaacttga acagactctc agaatacaag atatagaatt 3360
   gaaaagtgta acaagtaatt tgaatcaggt ttctcacact catgaaagtg aaaatgatct 3420
   ctttcatgaa aattgcatgt tgaaaaagga aattgccatg ctaaaactgg aagtagccac 3480
   actgaaacat caacaccagg tgaaggaaaa taaatacttt gaggacatta agattttaca 3540
   agaaaagaat gctgaacttc aaatgaccct aaaactgaaa cagaaaacag taacaaaaag 3600
   ggcatctcag tatagagagc agcttaaagt tctgacggca gagaacacga tgctgacttc 3660
   taaattgaag gaa                                                    3673
<210> 27
   <211> 1011
   <212> PRT
   <213> Homo sapiens
   <400> 27
   Met Val Ala Thr Leu Leu Ser Tyr Gly Ala Val Ile Glu Val Gln Asn
   1 5 10 15
   Lys Ala Ser Leu Thr Pro Leu Leu Leu Ala Ile Gln Lys Arg Ser Lys
   20 25 30
   Gln Thr Val Glu Phe Leu Leu Thr Lys Asn Ala Asn Ala Asn Ala Phe
   35 40 45
   Asn Glu Ser Lys Cys Thr Ala Leu Met Leu Ala Ile Cys Glu Gly Ser
   50 55 60
   Ser Glu Ile Val Gly Met Leu Leu Gln Gln Asn Val Asp Val Phe Ala
   65 70 75 80
   Glu Asp Ile His Gly Ile Thr Ala Glu Arg Tyr Ala Ala Ala Arg Gly
   85 90 95
   Val Asn Tyr Ile His Gln Gln Leu Leu Glu His Ile Arg Lys Leu Pro
   100 105 110
   Lys Asn Pro Gln Asn Thr Asn Pro Glu Gly Thr Ser Thr Gly Thr Pro
   115 120 125
   Asp Glu Ala Ala Pro Leu Ala Glu Arg Thr Pro Asp Thr Ala Glu Ser
   130 135 140
   Leu Leu Glu Lys Thr Pro Asp Glu Ala Ala Arg Leu Val Glu Gly Thr
   145 150 155 160
   Ser Ala Lys Ile Gln Cys Leu Gly Lys Ala Thr Ser Gly Lys Phe Glu
   165 170 175
   Gln Ser Thr Glu Glu Thr Pro Arg Lys Ile Leu Arg Pro Thr Lys Glu
   180 185 190
   Thr Ser Glu Lys Phe Ser Trp Pro Ala Lys Glu Arg Ser Arg Lys Ile
   195 200 205
   Thr Trp Glu Glu Lys Glu Thr Ser Val Lys Thr Glu Cys Val Ala Gly
   210 215 220
   Val Thr Pro Asn Lys Thr Glu Val Leu Glu Lys Gly Thr Ser Asn Met
   225 230 235 240
   Ile Ala Cys Pro Thr Lys Glu Thr Ser Thr Lys Ala Ser Thr Asn Val
   245 250 255
   Asp Val Ser Ser Val Glu Pro Ile Phe Ser Leu Phe Gly Thr Arg Thr
   260 265 270
   Ile Glu Asn Ser Gln Cys Thr Lys Val Glu Glu Asp Phe Asn Leu Ala
   275 280 285
   Thr Lys Ile Ile Ser Lys Ser Ala Ala Gln Asn Tyr Thr Cys Leu Pro
   290 295 300
   Asp Ala Thr Tyr Gln Lys Asp Ile Lys Thr Ile Asn His Lys Ile Glu
   305 310 315 320
   Asp Gln Met Phe Pro Ser Glu Ser Lys Arg Glu Glu Asp Glu Glu Tyr
   325 330 335
   Ser Trp Asp Ser Gly Ser Leu Phe Glu Ser Ser Ala Lys Thr Gln Val
   340 345 350
   Cys Ile Pro Glu Ser Met Tyr Gln Lys Val Met Glu Ile Asn Arg Glu
   355 360 365
   Val Glu Glu Leu Pro Glu Lys Pro Ser Ala Phe Lys Pro Ala Val Glu
   370 375 380
   Met Gln Lys Thr Val Pro Asn Lys Ala Phe Glu Leu Lys Asn Glu Gln
   385 390 395 400
   Thr Leu Arg Ala Ala Gln Met Phe Pro Ser Glu Ser Lys Gln Lys Asp
   405 410 415
   Asp Glu Glu Asn Ser Trp Asp Ser Glu Ser Pro Cys Glu Thr Val Ser
   420 425 430
   Gln Lys Asp Val Tyr Leu Pro Lys Ala Thr His Gln Lys Glu Phe Asp
   435 440 445
   Thr Leu Ser Gly Lys Leu Glu Glu Ser Pro Val Lys Asp Gly Leu Leu
   450 455 460
   Lys Pro Thr Cys Gly Arg Lys Val Ser Leu Pro Asn Lys Ala Leu Glu
   465 470 475 480
   Leu Lys Asp Arg Glu Thr Phe Lys Ala Glu Ser Pro Asp Lys Asp Gly
   485 490 495
   Leu Leu Lys Pro Thr Cys Gly Arg Lys Val Ser Leu Pro Asn Lys Ala
   500 505 510
   Leu Glu Leu Lys Asp Arg Glu Thr Leu Lys Ala Glu Ser Pro Asp Asn
   515 520 525
   Asp Gly Leu Leu Lys Pro Thr Cys Gly Arg Lys Val Ser Leu Pro Asn
   530 535 540
   Lys Ala Leu Glu Leu Lys Asp Arg Glu Thr Phe Lys Ala Ala Gln Met
   545 550 555 560
   Phe Pro Ser Glu Ser Lys Gln Lys Asp Asp Glu Glu Asn Ser Trp Asp
   565 570 575
   Phe Glu Ser Phe Leu Glu Thr Leu Leu Gln Asn Asp Val Cys Leu Pro
   580 585 590
   Lys Ala Thr His Gln Lys Glu Phe Asp Thr Leu Ser Gly Lys Leu Glu
   595 600 605
   Glu Ser Pro Asp Lys Asp Gly Leu Leu Lys Pro Thr Cys Gly Met Lys
   610 615 620
   Ile Ser Leu Pro Asn Lys Ala Leu Glu Leu Lys Asp Arg Glu Thr Phe
   625 630 635 640
   Lys Ala Glu Asp Val Ser Ser Val Glu Ser Thr Phe Ser Leu Phe Gly
   645 650 655
   Lys Pro Thr Thr Glu Asn Ser Gln Ser Thr Lys Val Glu Glu Asp Phe
   660 665 670
   Asn Leu Thr Thr Lys Glu Gly Ala Thr Lys Thr Val Thr Gly Gln Gln
   675 680 685
   Glu Arg Asp Ile Gly Ile Ile Glu Arg Ala Pro Gln Asp Gln Thr Asn
   690 695 700
   Lys Met Pro Thr Ser Glu Leu Gly Arg Lys Glu Asp Thr Lys Ser Thr
   705 710 715 720
   Ser Asp Ser Glu Ile Ile Ser Val Ser Asp Thr Gln Asn Tyr Glu Cys
   725 730 735
   Leu Pro Glu Ala Thr Tyr Gln Lys Glu Ile Lys Thr Thr Asn Gly Lys
   740 745 750
   Ile Glu Glu Ser Pro Glu Lys Pro Ser His Phe Glu Pro Ala Thr Glu
   755 760 765
   Met Gln Asn Ser Val Pro Asn Lys Gly Leu Glu Trp Lys Asn Lys Gln
   770 775 780
   Thr Leu Arg Ala Asp Ser Thr Thr Leu Ser Lys Ile Leu Asp Ala Leu
   785 790 795 800
   Pro Ser Cys Glu Arg Gly Arg Glu Leu Lys Lys Asp Asn Cys Glu Gln
   805 810 815
   Ile Thr Ala Lys Met Glu Gln Met Lys Asn Lys Phe Cys Val Leu Gln
   820 825 830
   Lys Glu Leu Ser Glu Ala Lys Glu Ile Lys Ser Gln Leu Glu Asn Gln
   835 840 845
   Lys Ala Lys Trp Glu Gln Glu Leu Cys Ser Val Arg Leu Pro Leu Asn
   850 855 860
   Gln Glu Glu Glu Lys Arg Arg Asn Val Asp Ile Leu Lys Glu Lys Ile
   865 870 875 880
   Arg Pro Glu Glu Gln Leu Arg Lys Lys Leu Glu Val Lys His Gln Leu
   885 890 895
   Glu Gln Thr Leu Arg Ile Gln Asp Ile Glu Leu Lys Ser Val Thr Ser
   900 905 910
   Asn Leu Asn Gln Val Ser His Thr His Glu Ser Glu Asn Asp Leu Phe
                   915                 920                 925
   His Glu Asn Cys Met Leu Lys Lys Glu Ile Ala Met Leu Lys Leu Glu
       930                 935                 940
   Val Ala Thr Leu Lys His Gln His Gln Val Lys Glu Asn Lys Tyr Phe
   945                 950                 955                 960
   Glu Asp Ile Lys Ile Leu Gln Glu Lys Asn Ala Glu Leu Gln Met Thr
                   965                 970                 975
   Leu Lys Leu Lys Gln Lys Thr Val Thr Lys Arg Ala Ser Gln Tyr Arg
               980                 985                 990
   Glu Gln Leu Lys Val Leu Thr Ala Glu Asn Thr Met Leu Thr Ser Lys
           995                 1000                1005
   Leu Lys Glu
       1010
<210> 28
   <211> 23
   <212> DNA
   <213> Homo sapiens
   <400> 28
   tctcatagat gctggtgctg atc 23
<210> 29
   <211> 24
   <212> DNA
   <213> Homo sapiens
   <400> 29
   cccagacatt gaattttggc agac 24
<210> 30
   <211> 56
   <212> PRT
   <213> Homo sapiens
   <400> 30
   Met Glu Glu Ile Ser Ala Ala Ala Val Lys Val Val Pro Gly Pro Glu
   1               5                   10                  15
   Arg Pro Ser Pro Phe Ser Gln Leu Val Tyr Thr Ser Asn Asp Ser Tyr
               20                  25                  30
   Ile Val His Ser Gly Asp Leu Arg Lys Ile His Lys Ala Ala Ser Arg
           35                  40                  45
   Gly Gln Val Arg Lys Leu Glu Lys
       50                  55